# EUROPEAN PATENT APPLICATION

(11) **EP 3 929 306 A1**
(43) Date of publication of application: **29.12.2021**
(21) Application number: 21174298.6
(22) Date of filing: 07.12.2017
(51) Int. Cl.: C12Q 1/6806, C12Q 1/6837

(54) **METHOD FOR ENRICHING TEMPLATE NUCLEIC ACIDS**

(30) Priority: 26.01.2017 EP 17153406
(62) Divisional of application: 17829138.1
(71) Applicant: QIAGEN GmbH, 40724 Hilden (DE)
(72) Inventor: GEIPEL, Andreas, 40724 Hilden (DE); KORFHAGE, Christian, 40724 Hilden (DE); REINECKE, Frank, 40724 Hilden (DE); JAGEMANN, Nadine, 40724 Hilden (DE)
(74) Representative: Friedrich, Rainer

(57) **Abstract**

The present invention provides new methods for enriching template nucleic acids on solid surface and for generating sequencing libraries which are particularly useful in molecular biology applications, such as next generation sequencing (NGS). The methods of the invention employ oligonucleotides bound to a solid surface comprising at least two functional sequence elements. The method is useful in applications where the density of the surface bound oligonucleotide is important/beneficial and/or where consecutive steps of an application require different sequences.

SEQUENCE LISTING

<110> Qiagen GmbH

<120> Method for enriching template nucleic acids

<130> Q1-7 EPDIV

<150> EP17153406.8
<151> 2017-01-26

<160> 12

<170> BiSSAP 1.3

<210> 1
<211> 76
<212> DNA
<213> Artificial Sequence

<220>
<223> Primer with two different sequence elements for specific
      capturing; immobilized onto surface

<400> 1
aaaaaaaaaa aaaaaaaaaa aaaaaaaaaa aaaaaccaac atcccacgcc tagtccccag     60

aacatgggct ttcttg                                                     76


<210> 2
<211> 19
<212> DNA
<213> Artificial Sequence

<220>
<223> Primer complementary to the first sequence element

<400> 2
ggactaggcg tgggatgtt                                                  19


<210> 3
<211> 20
<212> DNA
<213> Artificial Sequence

<220>
<223> qPCR-Primer for template 1

<400> 3
gcagagacaa gaggatggct                                                 20


<210> 4
<211> 19
<212> DNA
<213> Artificial Sequence

<220>
<223> qPCR-Primer for template 1

<400> 4
aacatcccac gcctagtcc                                                    19


<210> 5
<211> 20
<212> DNA
<213> Artificial Sequence

<220>
<223> qPCR-Primer for template 2

<400> 5
gccagattcc agatgaggac                                                   20


<210> 6
<211> 20
<212> DNA
<213> Artificial Sequence

<220>
<223> qPCR-Primer for template 2

<400> 6
ccagaacatg ggctttcttg                                                   20


<210> 7
<211> 171
<212> DNA
<213> Artificial Sequence

<220>
<223> Template that can be specifically captured by the first sequence
      element

<400> 7
caacaacaac aacaacaagg gcagagacaa gaggatggct aggcgaggag ctccagtcgg      60

ggggtgccca ggtcagtgga tcccctctcc accctggcct acctggtcgc catgggcgtg     120

cctgccaatg gtgatgggct tggtccagcc agggactagg cgtgggatgt t              171


<210> 8
<211> 126
<212> DNA
<213> Artificial Sequence

<220>
<223> Template that can be specifically captured by the second sequence
      element

<400> 8
caacaacaac aacaacaagg gccagattcc agatgaggac atcacagctt ccagtcagtg      60

gtcagagtcc acagctgcca aatatggaag gtgaggatgg ttacatcaag aaagcccatg     120

ttctgg                                                                126


<210> 9
<211> 220
<212> DNA
<213> Artificial Sequence

<220>
<223> Template that cannot be specifically captured by a sequence
      element

<400> 9
gagacgctgg agtacaaacg ccagctggct gcacttggcg acaaggttac gtatcaggag      60

cgcctgaacg cgctggcgca gcaggcggat aaattcgcac agcagcaacg ggcaaaacgg     120

gccgccattg atgcgaaaag ccgggggctg actgaccggc aggcagaacg ggaagccacg     180

gaacagcgcc tgaaggaaca gtatggcgat aatccgctgg                           220


<210> 10
<211> 60
<212> DNA
<213> Artificial Sequence

<220>
<223> Primer with to different sequence elements; immobilized onto
      surface

<400> 10
aaaaaaaaaa aaaaaaaaaa aaaaaaaaaa aaaaacccca gaacatgggc tttcttgccc      60


<210> 11
<211> 38
<212> DNA
<213> Artificial Sequence

<220>
<223> π-Amp-primer; immobilized onto surface

<400> 11
aaaaaaaaaa aaaaaaaaaa aaaaaaaaaa aaaaaccc                              38


<210> 12
<211> 59
<212> DNA
<213> Artificial Sequence

<220>

<223> Primer with to different sequence elements; immobilized onto
      surface

<400> 12
aaaaaaaaaa aaaaaaaaaa aaaaaaaaaa aaaaaccaac atcccacgcc tagtccccc       59

## Description

### FIELD OF THE INVENTION

The present invention provides new methods for enriching template nucleic acids and for generating sequencing libraries on solid surface which are particularly useful in molecular biology applications, such as next generation sequencing (NGS). The methods of the invention employ oligonucleotides bound to a solid surface comprising at least two functional sequence elements. The method is useful in applications where the density of the surface bound oligonucleotide is important/beneficial and/or where consecutive steps of an application require different sequences.

### BACKGROUND OF THE INVENTION

Next-generation sequencing (NGS), also known as high-throughput sequencing allows to acquire genome-wide data using highly parallel sequencing approaches for molecular biology applications, in vitro clinical diagnostics, or for forensics. Such applications include, e.g., de novo genome sequencing, transcriptome sequencing and epigenomics, as well as genetic screening for the identification of rare genetic variants and for efficient detection of either inherited or somatic mutations in cancer genes.

Hence, several sequencing platforms have been developed, which allow for low-cost, high-throughput sequencing. Such platforms include Illumina^{®} (Solexa), and Ion torrent: Proton / PGM by Life Technologies/ Thermo Fisher Scientific. NGS technologies, NGS platforms and common applications/fields for NGS technologies are e.g. reviewed in Voelkerding et al. (Clinical Chemistry 55:4 641-658, 2009) and Metzker (Nature Reviews/Genetics Volume 11, January 2010, pages 31-46).

Three main steps exist in NGS on most current platforms: preparation of the sample for high-throughput sequencing, enrichment of template nucleic acids on solid surface followed by an amplification step, and the actual sequencing.

Several technologies for enriching nucleic acids to be analysed prior to sequencing are known in the art.

In HaloPlex^{™} Target Enrichment System from Agilent Technologies (Schulz et al. 2012), the DNA of interest is digested with a mixture of different restriction enzymes. A library of biotinylated oligonucleotides is added that hybridize to both ends of the nucleic acid target sequences. The biotinylated oligonucleotides are captured together with the hybridized targets with streptavidin coated beads followed by amplification.

In the Agilent SureSelect target enrichment system (Van Vlierberghe et al. 2010, Fisher et al. 2011), after fragmentation and library preparation the target DNA fragments are hybridized to specific biotinylated RNA-oligonucleotides which are captured with streptavidin coated magnetic beads. After digestion of the RNA and washing, the targets are isolated.

In the NimbleGen technologies (Rui Chen et al. 2015), after fragmentation and library preparation the target DNA fragments are hybridized to specific oligonucleotides that are bound on surfaces or can be captured with beads to enrich the complex of target and capture oligonucleotide. The enriched fragment pool is amplified by PCR.

In the panel-PCR as applied in the GeneRead DNAseq Gene Panel System (Qiagen), the target fragments are directly amplified from the template nucleic acids in a multiplex PCR. The PCR-amplicons are then size-selectively purified and used for a library preparation.

Another method describes the addition of a second specific sequence element to oligonucleotides already bound to the surface for specific capturing of target sequences (Hopmans et al. 2014).

After enrichment of template nucleic acids, usually an amplification step is applied to generate identical copies of the templates. Several amplification technologies are known in the art.

For Clustered Amplification on beads (Porecca et al. 2006, Kim et al. 2007), the amplification of nucleic acid molecules takes place within emulsion vesicles. Each vesicle comprises a single target molecule, the amplification reagents and a small bead which is used as a solid support to immobilize amplified nucleic acid molecules to form the cluster. For the amplification two primers are necessary. One of them is within the solution, and the other primer is immobilized on the bead. Most often PCR is used for the amplification, but other amplification strategies may be adaptable to the method.

During Bridge Amplification (Adessi et al. 2000, US5641658, US6300070, US7790418, etc.), the amplification of nucleic acid molecules takes place if both primers are immobilized on solid support. Most often PCR is used for the amplification, but other amplification strategies may be adaptable to the method.

In Rolling Circle Amplification (US20020012933, US20030148344, Barbee et al. 2011, Nallur et al. 2001), the amplification reaction is started with circular nucleic acid molecules with one or two primers immobilized to the solid support.

During Clustered Amplification by template walking (Ma et al. 2013, US20120156728, US20140148345), which is also termed "Wildfire amplification" (WF), the amplification takes place by an isothermal amplification process with one primer within the solution and the other primer immobilized to the solid support. The amplification is started by an intermediated hybridization product which is formed by an immobilized primer that invades into a double-stranded target nucleic acid which is bound to the surface as well. The primer of this triple helix intermediate is elongated by a strand displacement polymerase so that a new double-stranded nucleic acid product and a single stranded nucleic acid product is formed. The single stranded nucleic acid product is used for primer hybridization and primer elongation to form a second double strand nucleic acid product.

During recombinase polymerase amplification (RPA) (Piepenburg et al., 2006), isothermal amplification of nucleic acid molecules is achieved by the binding of opposing oligonucleotide primers to template DNA and their extension by a DNA polymerase. The method makes use of proteins binding to single stranded primers.

In thermophilic Helicase-Dependent Amplification (tHDA), amplification takes place in an isothermal amplification process using two primers wherein DNA is separated using a helicase.

Another method is Isothermal Amplification on solid support, which is also termed "π-Amplification" (hereinafter referred to as "π-Amp"). The method enables amplification of nucleic acids which are immobilized to a surface to form discrete clusters of nucleic acids. The π-Amplification method comprises a) providing a surface comprising one or more immobilized primers attached to the surface, wherein the one or more immobilized primers comprise a blocking moiety at the 3'-end; b) providing one or more template nucleic acids under conditions suitable for hybridization of the one or more of the template nucleic acids to at least one of the immobilized primers, wherein one or more of the template nucleic acids hybridize to at least one of the immobilized primers; c) deblocking the immobilized primers hybridized to the template nucleic acid; and d) extending the deblocked immobilized primers to form a double stranded nucleic acid product comprising the template nucleic acid and a first strand product complementary to the template nucleic acid; e) after step d, providing a non-immobilized primer, wherein the non-immobilized primer is complementary to the 3'-end of the first strand product under conditions suitable for hybridization of the non-immobilized primer to the 3'-end of the first strand product; and f) extending the non-immobilized primer to form a second strand product that is complementary to the first strand product, wherein the extension of the non-immobilized primer displaces the template nucleic acid of the double stranded nucleic acid product formed in step d. During π-Amphfication, the extension of the immobilized primer results in production of a nucleic acid strand that is complementary to the template nucleic acid or a sequence identical to the template nucleic acid. The extended immobilized primer product, which is the first strand product or a nucleic acid identical to the first strand product such as the fourth strand product, can remain attached to the surface during the amplification process. The template nucleic acid and those sequences produced by the extension of the non-immobilized primer are able to move around the surface.

High efficiency hybridization during template nucleic acid enrichment is crucial in order to bind a sufficient portion of template nucleic acids to the solid surface to ensure sufficient library for sequencing. Thus, there is a need in the art for efficient enrichment methods that allow immobilization of template nucleic acids on a solid surface at a high density.

The methods of the invention allow highly efficient enrichment of template nucleic acids on solid surface by providing a high primer density on the solid surface.

A high primer density is important if a certain sequence or a certain group of sequences of a more complex mixture of sequences needs to be enriched on the surface. Advantageously, in the method of the invention, all the primers on the solid surface may comprise the same 3'-end required for an actual reaction step and all primers bound to the solid support expose the right sequence to their 3'-ends.

A high primer density may also be important if a portion of a complex mixture needs to be enriched via oligonucleotides with random sequences, thereby enabling generation of a complex sequencing library representing the complexity of the sample.

Further, high density of enriched template nucleic acids improves efficiency of next generation sequencing and allows for sequencing of template nucleic acids derived from different samples in parallel, thereby enabling more cost-effective sequencing.

If the captured nucleic acids need to be amplified on surface prior to analysis, a high density of functional primers is desired for the specific surface bound amplification.

The methods of the invention enable utilizing different functionalities within surface bound oligonucleotides for different reactions. Via a multi-stage mechanism the same oligonucleotides can provide different functionalities, while the overall primer density stays the same.

### SUMMARY OF THE INVENTION

The present invention relates to methods for enriching template nucleic acids and to methods for generating a sequencing library.

In particular, the present invention relates to methods for enriching template nucleic acids or for generating a sequencing library, wherein the method comprises:
a) hybridizing template nucleic acids to oligonucleotides bound to a solid surface and initially comprising at least two functional sequence elements;
b) extending the surface bound oligonucleotides hybridized to the template nucleic acids to form a double strand;
c) optionally modifying the double-stranded nucleic acids generated in step b);
d) 3' truncation of the surface bound oligonucleotides that have not been used in the template nucleic acid hybridization of step a); and
e) optionally modifying the single-stranded surface bound oligonucleotides generated in step d).

In some embodiments, the method of the invention further comprises the additional step of:
f) hybridizing further template nucleic acids to the surface bound oligonucleotides or
using functional sequence elements within surface bound oligonucleotides for a downstream application.

In some embodiments, the downstream application is nucleic acid amplification on the solid surface, de-coupling from solid surface, in-vitro transcription by the use of an RNA polymerase promotor within the oligonucleotide, labeling of immobilized nucleic acid by the use of a primer binding site or a molecular barcode region for identification within the oligonucleotide, sequencing, or a combination thereof.

In one embodiment, at least one of the functional sequence elements is a hybridization site or preferably a sequence useful for a downstream application.

In another embodiment, the functional sequence elements are consecutive or overlapping.

In certain embodiments, the functional sequence elements are separated by predefined cleavage sites or generated by hybridization of protecting oligonucleotides to the surface bound oligonucleotides.

In some embodiments, steps a) to c) are repeated at least once with template nucleic acids from different samples. In other embodiments, steps a) to e) are repeated at least once with template nucleic acids from the same sample or from different samples, optionally wherein the repetition(s) is (are) performed in parallel.

In some embodiments, the density of the surface bound oligonucleotides is between 500 - 500000 oligonucleotides / µm², more preferably 750 ― 200000 oligonucleotides / µm², most preferably 1000 - 100000 oligonucleotides / µm².

In other embodiments, the surface bound oligonucleotides comprise 2 to 20 functional sequence elements, more preferably 2 to 10, most preferably 2 to 5.

In certain embodiments, the length of the surface bound oligonucleotides is within the range of 4-200 nt, preferably 10-200 nt, more preferably 6-180 nt, more preferably 8-160 nt, more preferably 10-140 nt, most preferably 20-100 nt. In some embodiments, the length of the surface bound oligonucleotides is 10 nt, preferably 20 nt.

In some embodiments, all functional sequence elements of the same position within a surface bound oligonucleotide have a unique sequence or comprise 2-100000, preferably 2-50000, more preferably 2-25000, more preferably 2-10000, more preferably 2-5000, more preferably 2-2500, most preferably 2-1000 different sequences.

In certain embodiments, the 3' truncation is achieved enzymatically or chemically.

In some embodiments, the double-stranded nucleic acids bound to the surface are modified by introducing barcode sequences, adding sequencing adaptors, adding a fluorophore at the terminus, incorporation of modified bases, or other modifications.

In other embodiments, the single-stranded oligonucleotides bound to the surface are modified by adding biotin, labeling moieties, blocking moieties, or other modifications.

### BRIEF DESCRIPTION OF THE DRAWINGS

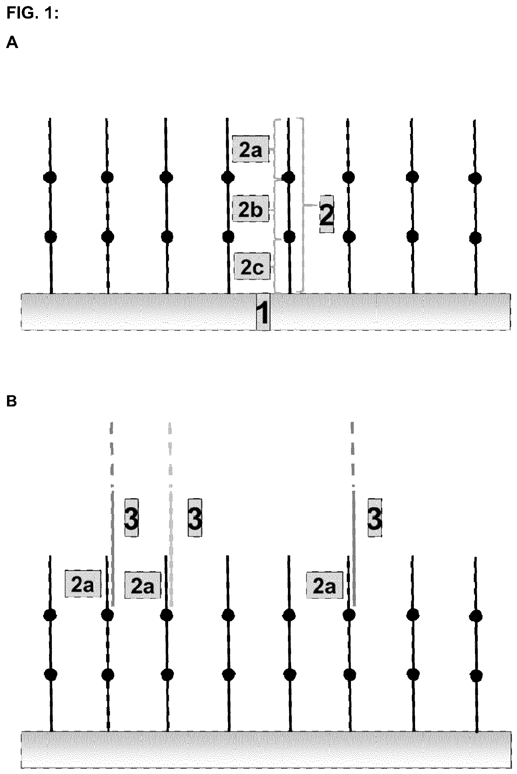

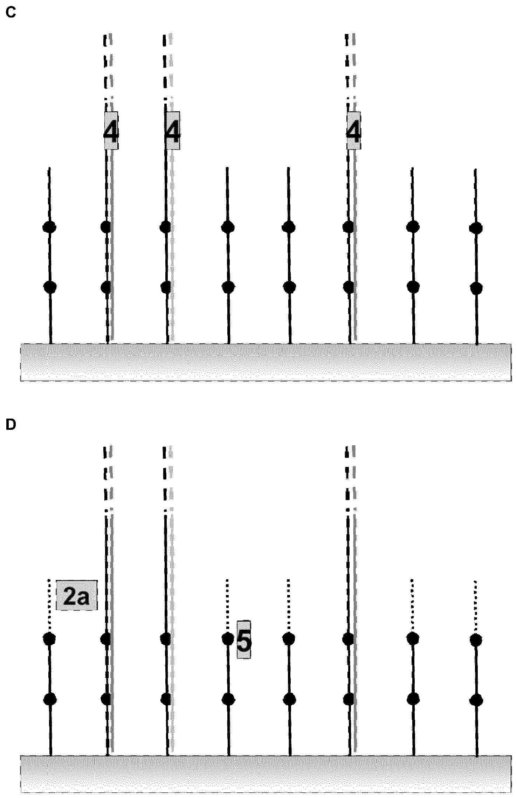

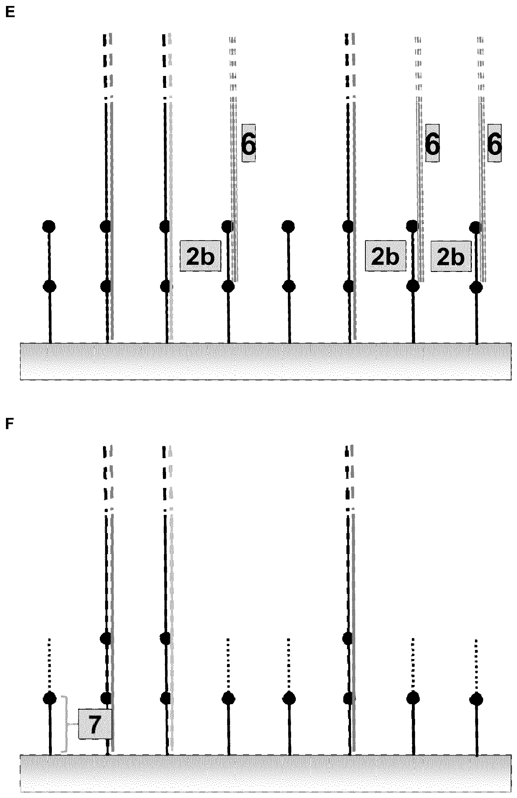

Figure 1: General method according to the invention.
   A) Oligonucleotides (2) with three consecutive sequence elements (2a, 2b, 2c) are immobilized on a solid support (1).
   B) Hybridization of template nucleic acid: The template nucleic acids (3) are hybridized to at least the most 3'-end sequence element (2a) of the oligonucleotides immobilized to solid support.
   C) Extension of oligonucleotides immobilized to the surface: The oligonucleotides are extended complementary to the template nucleic acid by a polymerase in 3'direction. A double strand product (4) is formed. Optionally, the double strands may be modified, e.g., by introducing barcode sequences, adding sequencing adaptors, adding a fluorophore at the terminus, incorporation of modified bases, or other modifications.
   D) Truncation of oligonucleotides not in use so far: The 3'-terminal sequence elements (2a) that are not in use so far are degraded (indicated by dashed lines) chemically or enzymatically. The degradation stops at the beginning (5) of the next sequence element (indicated by black dots).
   E) Optionally, steps B to D can be repeated, using the next sequence elements of the surface bound oligonucleotides (here 2b) to capture other targets (6).
   F) The last sequence element (7) may be a functional sequence for a downstream application, e.g., nucleic acid amplification on the solid surface, de-coupling from solid surface, in-vitro transcription by the use of an RNA polymerase promotor within the oligonucleotide, labeling of immobilized nucleic acid by the use of a primer binding site or a molecular barcode region for identification within the oligonucleotide, sequencing or another application, or a combination thereof.

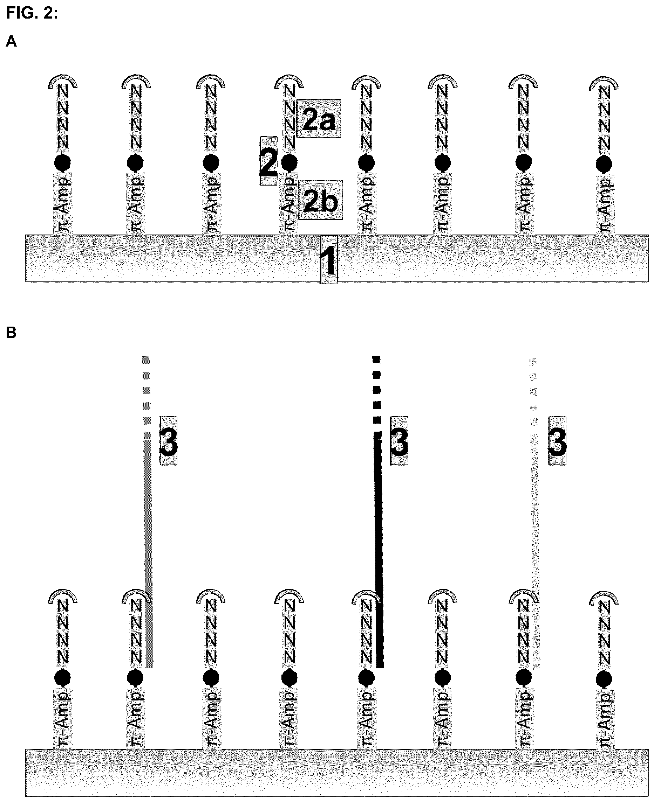

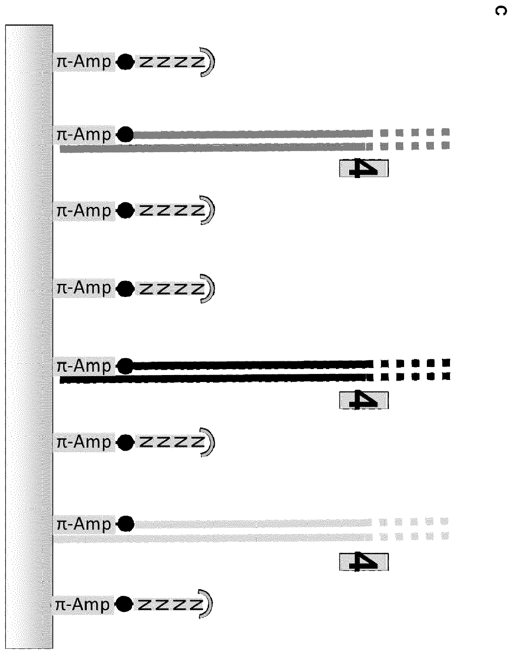

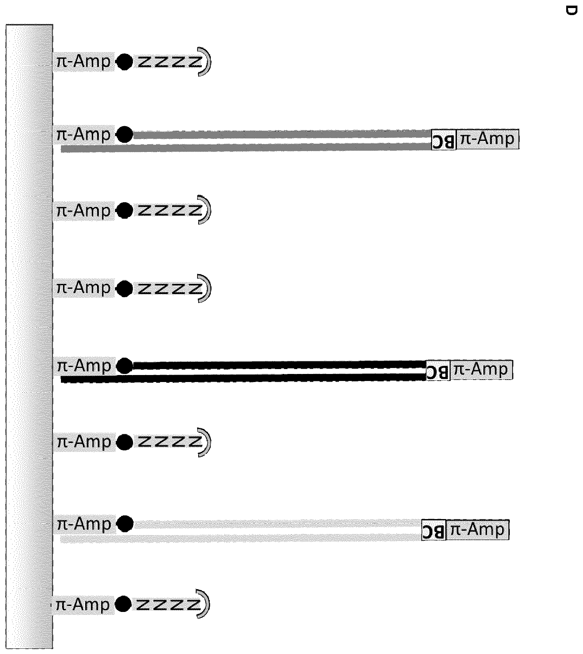

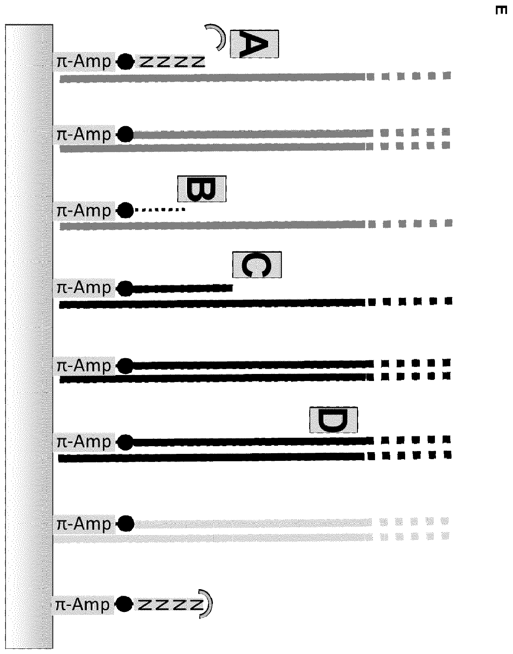

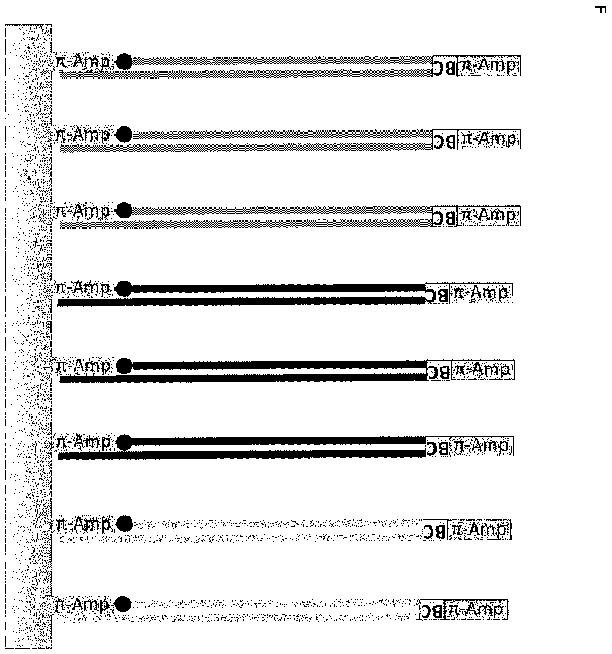

Figure 2: Capturing of sequences from a mixture with oligonucleotides comprising a random sequence and subsequent generation of NGS-library.
   A) Oligonucleotides (2) with a 3'-sequence element comprising a random sequence (2a) and functional sequences for π-Amp within the 5'-sequence element (2b) are immobilized on a solid support (1). The oligonucleotides are blocked with a dideoxy-CTP (indicated with bows), a modification that is needed for the π-Amp-reaction.
   B) Hybridization of template nucleic acid: Various template nucleic acids (3) of a complex nucleic acid sample hybridize to the fitting random 3'-terminal sequence elements of the oligonucleotides immobilized to solid support.
   C) Deblocking and extension of oligonucleotides immobilized to the surface: After hybridization, oligonucleotides bound to the surface are deblocked and extended complementary to the hybridized template nucleic acid strands by a polymerase in 5'-3' direction. A double strand product (4) is formed.
   D) Adaptor ligation: Adaptors are ligated to the free ends of the double strand DNA. These adaptors consist of the functional sequences needed for π-Amp at the 5'end and a barcode element at their 3'-end (BC).
   E) Simultaneous truncation of the 3'-terminal sequence elements and π-Amp-reaction: After hybridization of a displaced template strand (resulting from π-Amp-reaction) to the π-Amp sequence element (step A) of an oligonucleotide, degradation occurs of the dideoxy-CTP and the 3'-terminal sequence elements that are not complementary to the template (steps A and B). Degradation occurs enzymatically by a proofreading polymerase. The degradation stops at the region of complementarity between the template and the oligonucleotide. The extension of the oligonucleotide takes place (step C) (by the polymerase) and a new double strand product is formed (step D), which is a suitable substrate for another π-Amp-reaction.
   F) Amplification-product: The parallel reaction results in an amplified library in form of clusters on a surface. These clusters can be used thereafter for downstream experiments such as NGS.

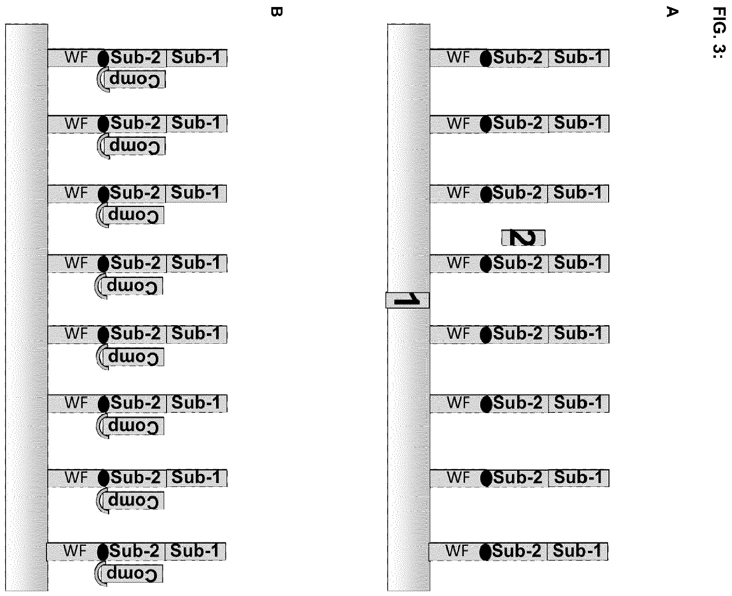

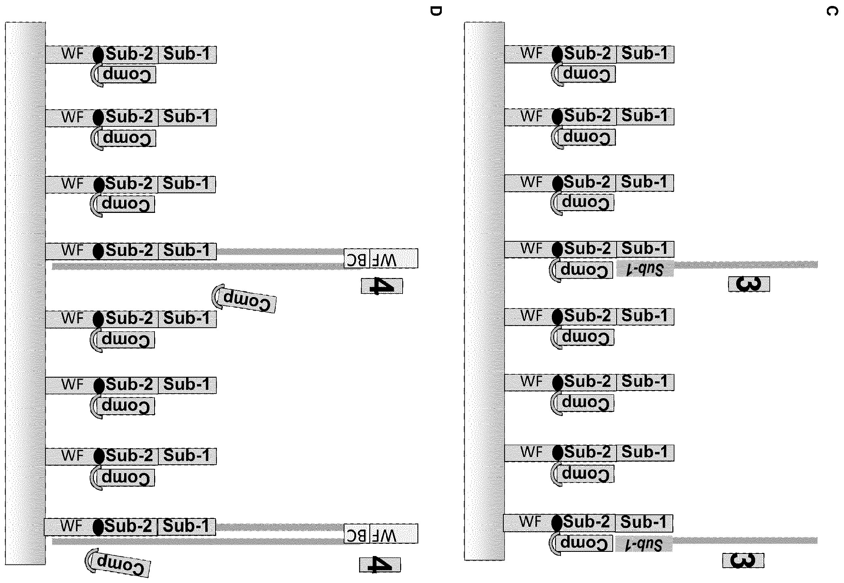

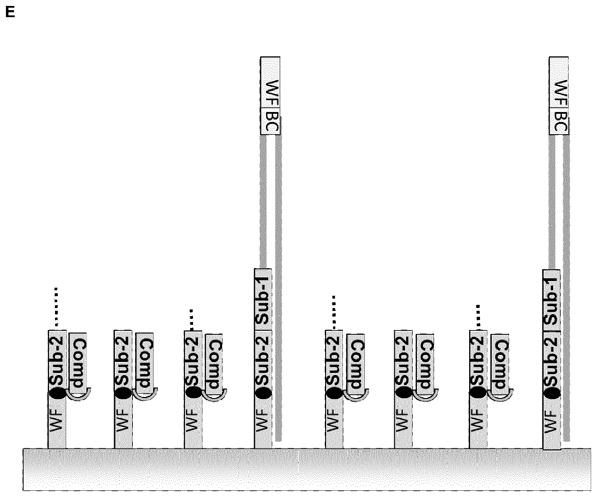

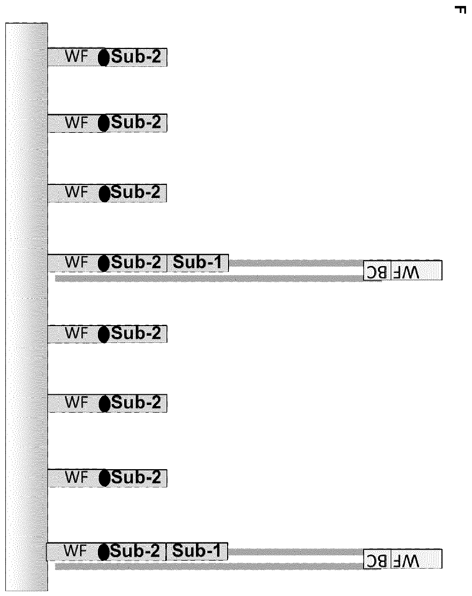

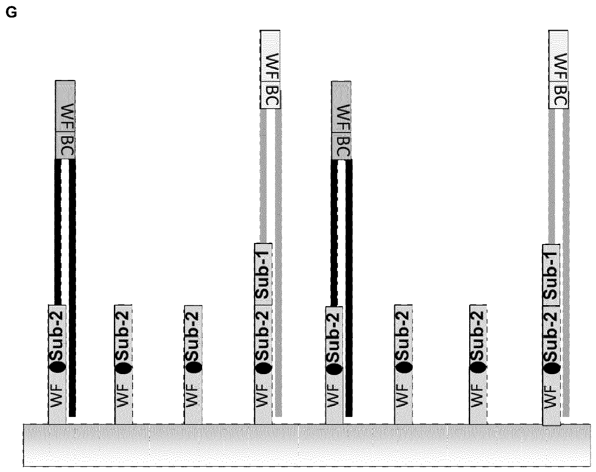

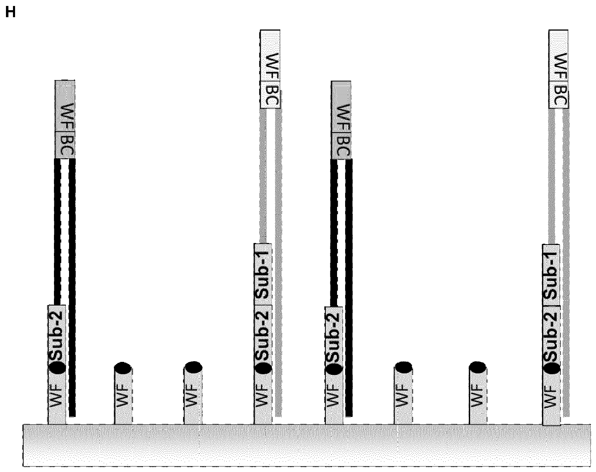

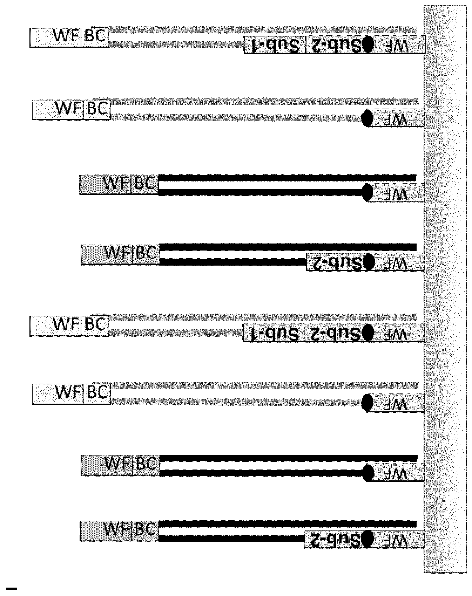

Figure 3: Capturing of two different specific subpopulations of a nucleic acid mixture and subsequent amplification:
   A) Immobilization of the oligonucleotides with multiple sequence elements:
      Oligonucleotides (2) with three consecutive sequence elements (Sub-1, Sub-2, WF) are immobilized on a solid support (1). The three consecutive sequence elements can be used for the binding of a first subpopulation 1 of a template nucleic acid complementary to sequence Sub-1 and a second subpopulation 2 of a template nucleic acid complementary to sequence Sub-2 and for a Wildfire-Amplification reaction (WF).
   B) Protection of sequence elements: An oligonucleotide (Comp) that is complementary to the sequence of the second sequence element (Sub-2) is hybridized to the oligonucleotide bound to the surface resulting in partially double stranded regions. In this example, this oligonucleotide (Comp) cannot be extended because the 3'-terminus is blocked by e.g. a dideoxy-nucleotide at the very 3'-end. Other modifications resulting in blocking the 3'-terminus are possible and are well known to a skilled person.
   C) Hybridization of a first subpopulation 1 of a template nucleic acid: The nucleic acids of subpopulation 1 (3) comprising a sequence complementary to Sub-1 (*Sub-1*) are hybridized specifically to the sequence element Sub-1 of the oligonucleotides immobilized to the solid support.
   D) Extension of the oligonucleotides: The oligonucleotides are extended complementary to the first template nucleic acid by a polymerase in 5'-3'direction. A double strand product is formed. The complementary oligonucleotide (Comp) is displaced by the polymerase during this process. Using a ligase, an adaptor sequence is added to the double stranded DNA (subpopulation 1). In this example, an adaptor (4) comprising a sequence element necessary for Wildfire amplification (WF) and Barcodes (BC) are ligated to the 5'-end.
   E) Truncation of the first 3'-terminal sequence element of the oligonucleotides bound to the surface: The 3'-sequence elements (Sub-1) of the non-hybridized surface bound oligonucleotides are degraded (indicated by black dotted lines), e.g., by a single strand specific exonuclease or a proof-reading polymerase. The degradation stops at the double stranded region formed by the complementary oligonucleotides (Comp). This double stranded region protects the sequence element Sub-2 from degradation.
   F) Denaturation of the oligonucleotides complementary to the sequence element Sub-2: The oligonucleotide (Comp) complementary to the sequence of the second sequence element (Sub-2) is denatured and aspirated. Denaturation may be performed by, e.g., changing temperature, pH or using denaturating reagents (e.g. Formamide, DMSO etc.).
   G) Hybridization of a second subpopulation 2 of a template nucleic acid: A second subpopulation 2 of a template nucleic acid from the same or another sample is specifically captured by the sequence element Sub-2 of the oligonucleotides bound to the surface. Using a ligase, adaptors are added to the ends of the double stranded DNA of the second subpopulation 2. In this example, an adaptor comprising a sequence element necessary for Wildfire amplification (WF) and Barcodes (BC) are ligated to the 5'-end.
   H) Truncation of the second 3'-teminal sequence element of the oligonucleotides bound to the surface: In this example, the second 3'-terminal sequence elements (Sub-2) of the oligonucleotides not in use so far are degraded enzymatically via a single strand specific exonuclease. In this example, the degradation stops at the phosphorothioate modification (indicated with the black ovals) that is positioned between the second (Sub-2) and the third (WF) sequence element of the oligonucleotides bound to the surface.
   I) Amplification: In this example, the Wildfire-reaction is performed, resulting in two independent amplified libraries in form of clusters on a surface. These clusters can be used thereafter for downstream experiments such as NGS.
Figure 4: Proof of specific functionality of the 3'-sequence element without functionality of the 5'-sequence element before the 3' truncation of the oligonucleotides immobilized to the surface as described in example 1. The graph shows the percentage of captured template 1 and 2.
Figure 5: Proof of activation of the function of the 5'-sequence element and deactivation of the function of the 3'-sequence element by 3' truncation of the oligonucleotides immobilized to the surface as described in example 2. The graph shows the percentage of captured template 1 and 2.
Figure 6: Proof that the functionalities of both sequence elements of the oligonucleotides immobilized to the surface can be used consecutively by the utilization of the 3' truncation as described in example 3. The graph shows the absolute amount of captured template 1 and 2.
Figure 7: Proof that an oligonucleotide immobilized to the surface can have (at least) one sequence element for capturing a template and another sequence element for amplifying the captured template. Proof that the amplification reaction and the 3' truncation can take place in the same reaction in parallel. Proof that a 3' truncation (to activate the Amplification sequence element) is needed for efficient amplification if no functional primers for the amplification are provided from the very beginning on. The graph shows the amplification factors for the indicated reaction conditions.

### DETAILED DESCRIPTION OF THE INVENTION

### Definitions

Unless defined otherwise, all technical and scientific terms used herein have the same meaning as commonly understood by one of ordinary skill in the art (e.g., in cell culture, molecular genetics, nucleic acid chemistry, hybridization techniques and biochemistry).

In practicing the present invention, many conventional techniques in molecular biology, microbiology, and recombinant DNA may be used. These techniques are well known and are explained in, for example, Current Protocols in Molecular Biology, Volumes I, II, and III, 1997 (F. M. Ausubel ed.); Sambrook et al., 1989, Molecular Cloning: A Laboratory Manual, Second Edition, Cold Spring Harbor Laboratory Press, Cold Spring Harbor, N.Y.; DNA Cloning: A Practical Approach, Volumes I and II, 1985 (D. N. Glover ed.); Oligonucleotide Synthesis, 1984 (M. L. Gait ed.); Nucleic Acid Hybridization, 1985, (Hames and Higgins); Transcription and Translation, 1984 (Hames and Higgins eds.); Animal Cell Culture, 1986 (R. I. Freshney ed.); Immobilized Cells and Enzymes, 1986 (IRL Press); Perbal, 1984, A Practical Guide to Molecular Cloning; the series, Methods In Enzymology (Academic Press, Inc.); Gene Transfer Vectors for Mammalian Cells, 1987 (J. H. Miller and M. P. Calos eds., Cold Spring Harbor Laboratory); and Methods in Enzymology Vol. 154 and Vol. 155 (Wu and Grossman, and Wu, eds., respectively). The terms "next generation sequencing" (NGS) and "high-throughput sequencing" are used as synonyms.

As used herein, the term "comprising" is to be construed as encompassing both "including" and "consisting of", both meanings being specifically intended, and hence individually disclosed embodiments in accordance with the present invention. "nt" is an abbreviation of "nucleotides".

The term "DNA" in the present invention relates to any one of viral DNA, prokaryotic DNA, archaeal DNA, and eukaryotic DNA. The DNA may also be obtained from any one of viral RNA, and mRNAfrom prokaryotes, archaea, and eukaryotes by generating complementary DNA (cDNA) by using a reverse transcriptase.

The terms "oligonucleotides" and "primers" are used as synonyms.

"Oligonucleotides" or "primers" are short DNA molecules. The length of the oligonucleotides may be within the range of 4-200 nt, preferably 6-180 nt, more preferably 8-160 nt, more preferably 10-140 nt, most preferably 20-100 nt. Other molecules are also possible that may partially substitute DNA with for example PNA or RNA. The primers may comprise the bases G, A, T, C or any other base like I, U, oxiG or other bases. The bases may be modified by organic moieties. The molecules can be single-stranded or double-stranded. The molecules may be linear or may comprise hairpin- or loop-structures. The molecules may comprise modifications such as biotin, labeling moieties, blocking moieties, or other modifications.

"Blocked oligonucleotides" are primers with blocked 3'-terminus. The blocking of the 3'terminus of oligonucleotides results in the inhibition of primer extension. Inhibition means that primer extension is blocked completely or is reduced significantly. This inhibition may be facilitated by blocks of phosphate, organic or inorganic moieties attached to the terminal nucleotide, dideoxy-nucleotides or nucleotides resulting in mispairing if hybridized to a complementary sequence.

A "solid support" is a surface which may be planar (e.g. planar chips or arrays) or curved (e.g. capillaries, beads). The support may be made of metal, glass, silica, plastics etc. It may also comprise a coated surface. The solid support may also comprise a soft and/or flexible surface or a hydrogel.

"Immobilization of oligonucleotides" refers to binding of oligonucleotides or primers to the solid support. Binding may be realized by, e.g., hybridization, streptavidin-biotin interaction, neutravidin-biotin interaction, complex-formation, covalent bonds or strong ionic bonds. Other methods of oligonucleotide immobilization are equally applicable. If immobilization is performed, a washing step may be necessary to get rid of unbound nucleic acids and undesired buffer components. The oligonucleotides (primers) are immobilized to the solid support so that the 5'-end of the oligonucleotides is directed to the surface and the 3'-end of the oligonucleotides is directed to the solution.

A "functional sequence element" is a section of an oligonucleotide that has one or more specific functions. The sequence element may include 5% to 95% of the complete oligonucleotide, depending on the overall number of sequence elements present in the oligonucleotide. Different sequence elements may partially overlap.

"Truncation of the surface bound oligonucleotides" means the reduction of the number of bases of the surface bound oligonucleotides so that the surface bound oligonucleotide still contains at least one functional sequence element after truncation. For example, the truncation can be achieved enzymatically with enzymes that possess a 3'-5'-directed exonuclease activity like exonucleases or proofreading polymerase. Examples for suitable enzymes are DNA-Polymerase I, Klenow polymerase, Phi29 Polymerase, Phusion Polymerase, T4 Polymerase, T7 Polymerase, Vent Polymerase, Deep vent Polymerase, TMA-Endonuclease, Exonuclease I, Uracil-N-Glycosylase (UDG or UNG), Apurinic/Apyrimidinic (AP) endonuclease, Endonuclease III, Endonuclease IV, Endonuclease V, Endonuclease VIII and others. The truncation can also be achieved chemically, for instance if oligonucleotides comprise different modifications that can be addressed via different chemical reactions. Truncation may be hindered, e.g., by hybridization of oligonucleotides complementary to sequence elements of the surface bound oligonucleotides or by chemical modifications (protecting oligonucleotides) or by chemical modifications within the surface bound oligonucleotides. The oligonucleotides consisting of different sequence elements may have chemical modifications at certain positions for preventing a complete truncation of the oligonucleotides, leading to a partial truncation stopping at the or in the vicinity of the chemical modification. This chemical modification may for example be a phosphorothioate that prevents the further digestion by many exonucleases or peptide nucleic acid backbones substituting the degradeable phosphate backbone of nucleic acids.

"Protecting oligonucleotides" are oligonucleotides for hindering of truncation of the surface bound oligonucleotides. The protecting oligonucleotides may be blocked or unblocked. The oligonucleotides may comprise the bases G, A, T, C or any other base like I, U, oxiG or other bases. The bases may be modified by organic moieties. The length of the oligonucleotides may be within the range 4-100 nt, preferably 6-80 nt, more preferably 8-60 nt, more preferably 10-50 nt, most preferably 20-40 nt. All oligonucleotides may have a unique sequence or comprise of 2, 3, 4, 5, 6, or more different sequences. The 5'-end of the oligonucleotides hybridizes to the very 3'-end of the sequence element of the surface bound oligonucleotide that shall be protected from truncation. The 3'-end may be variable.

The "template nucleic acid" is a nucleic acid molecule that is hybridized to at least one of the sequence elements of the surface bound oligonucleotides. The template nucleic acid may comprise one or more entities. If the template nucleic acid comprises more entities, the template nucleic acid may comprise one or more different sequences.

The template nucleic acid molecule comprises at least one hybridization site which is at least partially complementary to one of the sequence elements of the surface bound oligonucleotide. Such partially complementary nucleotide sequences can be ≥ 20 %, preferably ≥ 30 %, more preferably ≥ 50 %, more preferably ≥ 70 %, more preferably ≥ 80 %, more preferably ≥ 90 %, more preferably ≥ 95 %, or 100 % complementary to each other. The template nucleic acid molecules can either be artificially produced (e.g. by molecular or enzymatic manipulations or by synthesis) or may be a naturally occurring DNA or RNA. Artificially produced template nucleic acid molecules are nucleic acid molecules which were manipulated or produced in vitro, e.g. by involving the activities of ligases, polymerases, nucleases etc. Template nucleic acid molecules can also be isolated or purified from nature, i.e. from organisms or the environment, e.g. soil, water, air etc. Common methods for the isolation of naturally occurring nucleic acid molecules are well known to the skilled person. The template nucleic acid template can be subjected to the reaction mixture either directly after synthesis or isolation or after it has been purified. The purification can comprise one or several steps of physical, chemical, enzymatic purification, or another type.

"Hybridization" is a reaction where complementary or at least partially complementary nucleic acids (RNA, DNA, PNA or equivalent molecules) bind to each other in an antiparallel manner via hydrogen bounds. The resulting hybridization product is at least partially double-stranded. The hybridization reaction requires conditions well-known to the skilled person depending on the temperature, the pH value, the salt conditions, the concentration of the nucleic acid molecules in the reaction mixture, their lengths, GC contents, nucleotide sequences etc. The hybridization mixture may comprise reagents or enzymes that facilitate hybridization or strand exchange well known to the skilled person (e.g. recA).

"Polymerases" for extension of hybridized templates are enzymes which catalyse the covalent bonding between nucleotides. The polymerase can be heat labile or heat stabile. The polymerase may be a holoenzyme, a part of a holoenzyme, or a mutated or genetically modified form of polymerases from viruses, phages, prokaryotes, eukayrotes, or Archaebacteria. The polymerase may be isolated from the original organism or produced in genetically modified organism. Examples of polymerases are Phi 29-type DNA-polymerases, DNA-polymerase Klenow exo- and DNA-polymerase from Bacillus stearothermophilus (Bst DNA-polymerase), Taq-Polymerase, Pfu-Polymerase, Vent-Polymerase, T7-Polymerase, T4-Polymerase, DNA-Polymerase I-V,RNA-Polymerase I-III. Phi29-type DNA-polymerases are polymerases derived e.g. from the phages Phi 29, Cp-1, PRD-1, Phi 15, Phi 21, PZE, PZA, Nf, M2Y, B103, SF5, GA-1, Cp-5, Cp-7, PR4, PR5, PR722, and L 17. More examples for suitable polymerases are holoenzyme complexes from prokaryotes, eukaryotes, or Archaebacteria.

The term "PCR" refers to polymerase chain reaction, which is a standard method in molecular biology for DNA amplification.

The term "qPCR" refers to quantitative real-time PCR, a method used to amplify and simultaneously detect the amount of amplified target DNA molecule fragments. The process involves PCR to amplify one or more specific sequences in a DNA sample. At the same time, a detectable probe, typically a fluorescent probe, is included in the reaction mixture to provide real-time quantification. Two commonly used fluorescent probes for quantification of real-time PCR products are: (1) non-sequence-specific fluorescent dyes (e.g., SYBR^{®} Green) that intercalate into double-stranded DNA molecules in a sequence non-specific manner, and (2) sequence-specific DNA probes (e.g., oligonucleotides labeled with fluorescent reporters) that permit detection only after hybridization with the DNA targets or after incorporation into PCR products.

### Methods

The present invention refers to methods for enriching template nucleic acids and to methods for generating a sequencing library, e.g. prior to next generation sequencing.

In particular, the method referred to herein is characterized by 3' truncation of surface bound oligonucleotides comprising at least two functional sequence elements. The 3' truncation step enables utilization of different functionalities of consecutive sequence elements within surface bound oligonucleotides simultaneously or consecutively.

In some embodiments, the invention relates to methods for enriching template nucleic acids, wherein the method comprises:
a) hybridizing template nucleic acids to oligonucleotides bound to a solid surface and initially comprising at least two functional sequence elements;
b) extending the surface bound oligonucleotides hybridized to the template nucleic acids to form a double strand;
c) optionally modifying the double-stranded nucleic acids generated in step b);
d) 3' truncation of the surface bound oligonucleotides that have not been used in the template nucleic acid hybridization of step a); and
e) optionally modifying the single-stranded surface bound oligonucleotides generated in step d).

In other embodiments, the present invention relates to methods for generating a sequencing library, wherein the method comprises:
a) hybridizing template nucleic acids to oligonucleotides bound to a solid surface and initially comprising at least two functional sequence elements;
b) extending the surface bound oligonucleotides hybridized to the template nucleic acids to form a double strand;
c) optionally modifying the double-stranded nucleic acids generated in step b);
d) 3' truncation of the surface bound oligonucleotides that have not been used in the template nucleic acid hybridization of step a); and
e) optionally modifying the single-stranded surface bound oligonucleotides generated in step d).

In the method of the invention, the oligonucleotides are immobilized to the solid support via their 5'-end.

In certain embodiments, the method further comprises removing unbound template nucleic acids after hybridization step a), preferably by a washing step.

In some embodiments, the method further comprises the additional step of
f) hybridizing further template nucleic acids to the surface bound oligonucleotides or
using functional sequence elements within surface bound oligonucleotides for a downstream application.

Downstream applications according to the invention may comprise nucleic acid amplification on the solid surface, de-coupling from solid surface, in-vitro transcription by the use of an RNA polymerase promotor within the oligonucleotide, labeling of immobilized nucleic acid by the use of a primer binding site or a molecular barcode region for identification within the oligonucleotide, sequencing, or a combination thereof. Amplification methods may comprise amplification technologies known in the art. De-coupling from solid surface may be performed, e.g., by the use of a restriction site within the oligonucleotide, elution under denaturing conditions, disrupting the surface, specifically eluting the nucleic acids.

In a preferred embodiment, in the method of the invention, at least one of the functional sequence elements is a hybridization site or preferably a sequence useful for a downstream application.

In some embodiments, in the method of the invention, the functional sequence elements are consecutive or overlapping. In some preferred embodiments, the functional sequence elements are separated by predefined cleavage sites or generated by hybridization of protecting oligonucleotides to the surface bound oligonucleotides.

In a preferred embodiment, in the method of the invention, steps a) to c) are repeated at least once with template nucleic acids from different samples. For instance, when template nucleic acids from different samples are to be sequenced, steps a) to c) may be repeated with every single sample. Step c) of each repetition may be used to introduce modifications to distinguish template nucleic acids derived from different samples from each other, e.g., by introducing sample-specific barcode sequences. Finally, 3' truncation of step d) may expose functional sequence elements within surface bound oligonucleotides necessary for a downstream application. For instance, subsequent 3' truncation step d) may expose functional sequence elements useful for amplification, thereby enabling simultaneous amplification of template nucleic acids derived from different samples.

In the method of the invention, 3' truncation of the surface bound oligonucleotides may be repeated up to (n-1) times with n being the total number of functional sequence elements present within the oligonucleotide. In some embodiments, 3' truncation takes place at the most 3' sequence element of the surface bound oligonucleotides.

In another preferred embodiment, in the method of the invention, steps a) to e) are repeated at least once with template nucleic acids from the same sample or from different samples. For instance, the 3' sequence element initially exposed at the 3'-end of surface bound oligonucleotides may enable capturing of a first subpopulation of template nucleic acids complementary to said sequence element. Upon 3' truncation, the subsequently exposed sequence element allows binding of a second subpopulation of template nucleic acids complementary to this sequence element. Accordingly, consecutive functional sequence elements may be exposed upon different 3' truncation steps, thereby enabling binding of specific subpopulations of nucleic acids. In a preferred embodiment, the repetition(s), i.e. steps a) to e), is (are) performed in parallel. In this way, different functions of the oligonucleotide immobilized to the solid surface can be utilized in one reaction.

In the method of the invention, after the first 3' truncation step of the surface bound oligonucleotides, the truncated oligonucleotide still contains at least one functional sequence element.

In some embodiments, in the method of the invention, the density of the surface bound oligonucleotides is between 500 - 500000 oligonucleotides / µm², more preferably 750 - 200000 oligonucleotides / µm², most preferably 1000 - 100000 oligonucleotides / µm².

In some embodiments, the surface bound oligonucleotides comprise 2 to 20 functional sequence elements, more preferably 2 to 10, most preferably 2 to 5.

In some embodiments, the length of the surface bound oligonucleotides is within the range of 4-200 nt, preferably 10-200 nt, more preferably 6-180 nt, more preferably 8-160 nt, more preferably 10-140 nt, most preferably 20-100 nt. In some embodiments, the length of the surface bound oligonucleotides is 10 nt, preferably 20 nt.

In some embodiments, all functional sequence elements of the same position within a surface bound oligonucleotide have a unique sequence. For instance, surface bound oligonucleotides may comprise a first sequence element complementary to a first subpopulation of template nucleic acids and further sequence elements complementary to other subpopulations of template nucleic acids, thereby enabling enrichment of specific template nucleic acids. Different functional sequence elements may be exposed simultaneously or consecutively. Surface bound oligonucleotides may additionally comprise sequence elements useful for downstream applications.

In other embodiments, all functional sequence elements of the same position within a surface bound oligonucleotide comprise 2-100000, preferably 2-50000, more preferably 2-25000, more preferably 2-10000, more preferably 2-5000, more preferably 2-2500, most preferably 2-1000 different sequences. Alternatively, the sequence elements comprise random sequences allowing capturing of a variety of template nucleic acids out of a complex mixture of nucleic acids.

In some embodiments, in the method of the invention, the 3' truncation is achieved enzymatically or chemically.

In some embodiments, the double-stranded nucleic acids bound to the surface are modified by introducing barcode sequences, adding sequencing adaptors, adding a fluorophore at the terminus, incorporation of modified bases, or other modifications.

In other embodiments, the single-stranded oligonucleotides bound to the surface are modified by adding biotin, labeling moieties, blocking moieties, or other modifications.

In a preferred embodiment, a panel of specific nucleic acids is captured followed by adaptor ligation (incl. barcode) and amplified for a subsequent NGS experiment according to the following protocol:
1. Providing a solid support comprising oligonucleotides with two consecutive sequence elements.
   a. For example, immobilization of oligonucleotides can be achieved as step of the method via covalent binding or streptavidin/avidin- biotin interaction. Other methods of oligonucleotide immobilization are equally applicable. If immobilization is performed, a washing step may be necessary to get rid of unbound nucleic acids and undesired buffer components.
   b. The 3'-elements of the oligonucleotides contain sequences that are complementary to sequences of the nucleic acids to be captured.
   c. The 5'-sequence element of the oligonucleotides contains the functional sequence element, needed for the amplification process at the very 5'-end, followed in 3'-direction by the adapter sequence for the desired downstream application (e.g. next generation sequencing), followed in 3'-direction by a molecular barcode region for identification and compensation of possible amplification bias during the amplification process.
2. Providing a complex nucleic acid mixture that contains the nucleic acids of interest in a buffer appropriate for specific hybridization.
3. Specific hybridization of the nucleic acids to the complementary 3'-elements of the oligonucleotides bound to the solid support.
4. Addition of polymerase for extension of the immobilized oligonucleotides complementary to the captured template nucleic acid in 3'-direction. A double strand product is formed.
   a. Alternatively, the buffer for hybridization contains a polymerase and desoxyribonucleotides for extension of the immobilized oligonucleotides complementary to the captured template nucleic acid in 3'-direction. A double strand product is formed.
   b. A washing step may be necessary to get rid of unbound nucleic acids and undesired buffer components.
5. Adaptor ligation of the double stranded extension product by using an appropriate buffer, a double strand specific ligase and an adapter-oligonucleotide. The adapter sequence contains the sequence needed for the successive amplification process, followed in 3'-direction by the sequence needed for the downstream application (next generation sequencing) followed in 3'-direction by a sample barcode sequence.
   a. Alternatively the adapter sequence only contains the sequence needed for the amplification process and an oligonucleotide of the downstream amplification method introduces the barcode sequence and the sequence needed for the downstream application in form of a 5'-tail.
   b. A washing step may be necessary to get rid of unbound nucleic acids and undesired buffer components.
6. An amplification step of captured products from step 5 is performed by providing the appropriate buffer, enzymes and desoxyribonucleotides.
   a. Typical amplification methods are Wildfire-amplification, π-amplification, Bridge-amplification, recombinase polymerase based amplification processes such as RPA or tHDA, PCR-based amplification.
   b. For the amplification process a 3'-5'exonuclease activity (e.g. proofreading activity of a polymerase) is needed to degrade the 3'-elements of the so far unused immobilized oligonucleotides in order to activate the functionality of the 5'-elements (providing the functional sequence for the amplification process).
   c. A washing step may be necessary to get rid of unbound nucleic acids and undesired buffer components.
7. The captured, amplified and barcoded panel sequences are eluted from the surface.
   a. For example, elution can be performed by using denaturing conditions, disrupting the surface, specifically eluting the nucleic acids.
   b. In another example, elution is performed by using an enzymatic activity (e.g. restriction enzyme, Uracil-N-glycosylase)
   c. The resulting population of DNA molecules comprises all sequence elements desired for the downstream application on both ends (adapter sequences), a molecular barcode and a sample barcode and can be directly used.

The advantages of the above described embodiment are the following:
1. A first adapter sequence including a molecular barcode and other functional elements needed for downstream applications (e.g. amplification) is introduced into the nucleic acids during the capturing process. The introduction of the molecular barcode does not interfere with the amplification process.
2. A second adapter sequence has to be ligated to the target nucleic acids. Therefore only one of the two adaptors is ligated to the template.
3. The process prevents the ligation of the same adapter sequence at both ends of the nucleic acids and the amplification of such nucleic acids during the amplification process, a common problem in nucleic acid preparation for sequencing.
4. A maximum density of surface bound oligonucleotides can be used for capturing of target nucleic acids due to the dual function of these oligonucleotides
5. A maximum density of surface bound oligonucleotides can be used for amplification of captured target nucleic acids due to the dual function of these oligonucleotides.

In another preferred embodiment, a random fraction of a complex mixture of nucleic acids is captured followed by adaptor ligation (incl. barcode) and amplified for a subsequent NGS experiment according to the following protocol:
1. Providing a solid support comprising oligonucleotides with two consecutive sequence elements.
   a. For example, immobilization of oligonucleotides can be achieved as step of the method via covalent binding or streptavidin/avidin- biotin interaction. Other methods of oligonucleotide immobilization are equally applicable. If immobilization is performed, a washing step may be necessary to get rid of unbound nucleic acids and undesired buffer components.
   b. The 3'-elements of the oligonucleotides contain sequences that are partially or completely random in order to capture a random fraction of nucleic acids from the complex mixture of nucleic acids.
   c. The 5'-sequence element of the oligonucleotides contains the functional sequence element, needed for the amplification process at the very 5'-end, followed in 3'-direction by the adapter sequence for the desired downstream application (e.g. next generation sequencing), followed in 3'-direction by a molecular barcode region for identification and compensation of possible amplification bias during the amplification process.
2. Providing a complex nucleic acid mixture that contains the nucleic acids of interest in a buffer appropriate for hybridization.
3. Hybridization of the nucleic acids to the complementary 3'-elements of the oligonucleotides bound to the solid support.
4. Addition of polymerase for extension of the immobilized oligonucleotides complementary to the captured template nucleic acid in 3'-direction. A double strand product is formed
   a. Alternatively, the buffer for hybridization contains a polymerase and desoxyribonucleotides for extension of the immobilized oligonucleotides complementary to the captured template nucleic acid in 3'-direction. A double strand product is formed.
   b. A washing step may be necessary to get rid of unbound nucleic acids and undesired buffer components.
5. Adaptor ligation of the double stranded extension product by using an appropriate buffer, a double strand specific ligase and an adapter-oligonucleotide. The adapter sequence contains the sequence needed for the successive amplification process, followed in 3'-direction by the sequence needed for the downstream application (next generation sequencing) followed in 3'-direction by a sample barcode sequence.
   a. Alternatively the adapter sequence only contains the sequence needed for the amplification process and an oligonucleotide of the downstream amplification method introduces the barcode sequence and the sequence needed for downstream application in form of a 5'-tail.
   b. A washing step may be necessary to get rid of unbound nucleic acids and undesired buffer components.
6. An amplification step of captured products from step 5 is performed by providing the appropriate buffer, enzymes and desoxyribonucleotides.
   a. Typical amplification methods are Wildfire-amplification, π-amplification, Bridge-amplification, recombinase polymerase based amplification processes such as RPA or tHDA, PCR-based amplification.
   b. For the amplification process a 3'-5'exonuclease activity (e.g. proofreading activity of a polymerase) is needed to degrade the 3'-elements of the so far unused immobilized oligonucleotides in order to activate the functionality of the 5'-elements (providing the functional sequence for the amplification process).
   c. A washing step may be necessary to get rid of unbound nucleic acids and undesired buffer components.
7. The captured, amplified and barcoded panel sequences are eluted from the surface.
   a. For example, elution can be performed by using denaturing conditions, disrupting the surface, specifically eluting the nucleic acids.
   b. In another example, elution is performed by using an enzymatic activity (e.g. restriction enzyme, Uracil-N-glycosylase)
   c. The resulting population of DNA molecules comprises all sequence elements desired for the downstream application on both ends (adapter sequences), a molecular barcode and a sample barcode and can be directly used.

The advantages of the above described embodiment are the following:
1. A first adapter sequence including a molecular barcode and other functional elements needed for downstream applications (e.g. amplification) is introduced into the nucleic acids during the capturing process. The introduction of the molecular barcode does not interfere with the amplification process.
2. A second adapter sequence has to be ligated to the target nucleic acids. Therefore only one of the two adaptors is ligated to the template.
3. The process prevents the ligation of the same adapter sequence at both ends of the nucleic acids and the amplification of such nucleic acids during the amplification process, a common problem in nucleic acid preparation for sequencing.
4. A maximum density of surface bound oligonucleotides can be used for capturing of target nucleic acids due to the dual function of these oligonucleotides
5. A maximum density of surface bound oligonucleotides can be used for amplification of captured nucleic acids due to the dual function of these oligonucleotides

In a particularly preferred embodiment, one or several panels of specific nucleic acids are captured followed by adaptor ligation (incl. barcode), amplification and direct sequencing, according to the following protocol:
1. Providing a solid support comprising oligonucleotides with two consecutive sequence elements.
   a. For example, immobilization of oligonucleotides can be achieved as step of the method via covalent binding or streptavidin/avidin- biotin interaction. Other methods of oligonucleotide immobilization are equally applicable. If immobilization is performed, a washing step may be necessary to get rid of unbound nucleic acids and undesired buffer components.
   b. The 3'-elements of the oligonucleotides contain sequences that are complementary to sequences of the nucleic acids to be captured.
   c. The 5'-sequence element of the oligonucleotides contains the functional sequence element, needed for the amplification process at the very 5'-end, followed in 3'- direction by the sequencing primer binding site, followed in 3'direction by a molecular barcode region for identification and compensation of possible amplification bias during the amplification process.
2. Providing a complex nucleic acid mixture that contains the nucleic acids of interest in a buffer appropriate for specific hybridization.
3. Specific hybridization of the nucleic acids to the complementary 3'-elements of the oligonucleotides bound to the solid support.
4. Addition of polymerase for extension of the immobilized oligonucleotides complementary to the captured template nucleic acid in 3'-direction. A double strand product is formed.
   a. Alternatively, the buffer for hybridization contains a polymerase and desoxyribonucleotides for extension of the immobilized oligonucleotides complementary to the captured template nucleic acid in 3'-direction. A double strand product is formed.
   b. A washing step may be necessary to get rid of unbound nucleic acids and undesired buffer components.
5. Adaptor ligation of the double stranded extension product by using an appropriate buffer, a double strand specific ligase and an adapter-oligonucleotide. The adapter sequence contains the sequence needed for the successive amplification process, followed in 3'-direction by the sequencing primer binding site, followed in 3'-direction by a sample barcode sequence.
   a. Alternatively, the adapter sequence only contains the sequence needed for the amplification process and an oligonucleotide of the downstream amplification method introduces the barcode sequence and the sequence needed for the downstream application in form of a 5'-tail.
   b. A washing step may be necessary to get rid of unbound nucleic acids and undesired buffer components.
   Optionally: After adaptor ligation and a possible washing step, steps 2 to 5 (the capturing) can be repeated with the same or another complex nucleic acid mixture considering the following changes:
   a. The surface bound oligonucleotides comprise several consecutive sequence elements for capturing of different target nucleic acids, following the 5'sequence element for amplification and sequencing in 3'-direction. The number of such sequence elements limits the number of times the capturing process can be repeated. The order of these elements is reverse to the order of capturing steps (in the first capturing process the most 3'-sequence element is used for capturing, followed by the next sequence element in 5'direction for the second capturing and so on)
   b. After step 5 of the protocol, the unused most 3'-sequence elements for capturing are digested using a single strand specific 3'-5'exonuclease activity. In order to protect digestion of further sequence elements during this process, the next sequence elements are protected by a prior specific hybridization of an oligonucleotide complementary to these sequence elements. Alternatively the next sequence elements are protected from digestion by an intrinsic property of the oligonucleotides (for example a thiophosphate backbone, resistant to digestion by the enzyme)
   c. After digestion the protecting oligonucleotides are washed away using a denaturing buffer with denaturing conditions like high pH, denaturing agents like DMSO or formamide, high temperature, low salt conditions or a combination of these factors.
   d. The adapter ligation introduces the sample barcode, which is unique for each capturing process.
6. An amplification step of captured products from step 5 is performed by providing the appropriate buffer, enzymes and desoxyribonucleotides.
   a. Typical amplification methods are Wildfire-amplification, π-amplification, Bridge-amplification, recombinase polymerase based amplification processes such as RPA or tHDA, PCR-based amplification.
   b. For the amplification process a 3'-5'exonuclease activity (e.g. proofreading activity of a polymerase) is needed to degrade the 3'-elements of the so far unused immobilized oligonucleotides in order to activate the functionality of the 5'-elements (providing the functional sequence for the amplification process).
   c. A washing step may be necessary to get rid of unbound nucleic acids and undesired buffer components.
7. The captured, amplified and barcoded panel sequences are directly used in a sequencing experiment.
   a. The resulting population of DNA molecules comprises all sequence elements desired for the downstream application on both ends (primer binding sites, barcode sequences) is present on the surface in form of discrete clusters and can be directly used.
   b. All sequencing reactions using discrete nucleic acid clusters on a solid surface for sequencing can be used.

The advantages of the above described embodiment are the following:
1. A first adapter sequence including a molecular barcode and other functional elements needed for downstream applications (e.g. amplification) is introduced into the nucleic acids during the capturing process. The introduction of the molecular barcode does not interfere with the amplification process.
2. A second adapter sequence has to be ligated to the target nucleic acids. Therefore only one of the two adaptors is ligated to the template.
3. The process prevents the ligation of the same adapter sequence at both ends of the nucleic acids and the amplification of such nucleic acids during the amplification process, a common problem in nucleic acid preparation for sequencing.
4. A maximum density of surface bound oligonucleotides can be used for capturing of target nucleic acids due to the dual function of these oligonucleotides
5. A maximum density of surface bound oligonucleotides can be used for amplification of captured target nucleic acids due to the dual function of these oligonucleotides
6. The captured and amplified sequences are forming clusters that can be directly used for sequencing on the same surface.
7. Several capturing processes can be done on one surface to accumulate different compositions of target sequences with different sample barcodes for differentiation. This way the full capacity of the surface can be used even if the efficiency of a single capturing process is low.

In another preferred embodiment, a random fraction of one or several complex mixtures of nucleic acids is captured followed by adaptor ligation (incl. barcode), amplification and direct sequencing, according to the following protocol:
1. Providing a solid support comprising oligonucleotides with two consecutive sequence elements.
   a. For example, immobilization of oligonucleotides can be achieved as step of the method via covalent binding or streptavidin/avidin- biotin interaction. Other methods of oligonucleotide immobilization are equally applicable. If immobilization is performed, a washing step may be necessary to get rid of unbound nucleic acids and undesired buffer components.
   b. The 3'-elements of the oligonucleotides contain sequences that are partially or completely random in order to capture a random fraction of nucleic acids from the complex mixture of nucleic acids.
   c. The 5'-sequence element of the oligonucleotides contains the functional sequence element, needed for the amplification process at the very 5'-end, followed in 3'- direction by the sequencing primer binding site, followed in 3'direction by a molecular barcode region for identification and compensation of possible amplification bias during the amplification process.
2. Providing a complex nucleic acid mixture that contains the nucleic acids of interest in a buffer appropriate for hybridization.
3. Hybridization of the nucleic acids to the complementary 3'-elements of the oligonucleotides bound to the solid support.
4. Addition of polymerase for extension of the immobilized oligonucleotides complementary to the captured template nucleic acid in 3'-direction. A double strand product is formed
   a. Alternatively, the buffer for hybridization contains a polymerase and desoxyribonucleotides for extension of the immobilized oligonucleotides complementary to the captured template nucleic acid in 3'-direction. A double strand product is formed.
   b. A washing step may be necessary to get rid of unbound nucleic acids and undesired buffer components.
5. Adaptor ligation of the double stranded extension product by using an appropriate buffer, a double strand specific ligase and an adapter-oligonucleotide. The adapter sequence contains the sequence needed for the successive amplification process, followed in 3'-direction by the sequencing primer binding site, followed in 3'-direction by a sample barcode sequence.
   a. Alternatively the adapter sequence only contains the sequence needed for the amplification process and an oligonucleotide of the downstream amplification method introduces the barcode sequence and the sequence needed for the downstream application in form of a 5'-tail.
   b. A washing step may be necessary to get rid of unbound nucleic acids and undesired buffer components.
   Optionally: After adaptor ligation and a possible washing step, steps 2 to 5 (the capturing) can be repeated with another complex nucleic acid mixture considering the following changes:
   a. The adapter ligation introduces the sample barcode, which is unique for each capturing process.
6. An amplification step of captured products from step 5 is performed by providing the appropriate buffer, enzymes and desoxyribonucleotides.
   a. Typical amplification methods are Wildfire-amplification, π-amplification, Bridge-amplification, recombinase polymerase based amplification processes such as RPA or tHDA, PCR-based amplification.
   b. For the amplification process a 3'-5'exonuclease activity (e.g. proofreading activity of a polymerase) is needed to degrade the 3'-elements of the so far unused immobilized oligonucleotides in order to activate the functionality of the 5'-elements (providing the functional sequence for the amplification process).
   c. A washing step may be necessary to get rid of unbound nucleic acids and undesired buffer components.
7. The captured, amplified and barcoded panel sequences are directly used in a sequencing experiment.
   a. The resulting population of DNA molecules comprises all sequence elements desired for the downstream application on both ends (primer binding sites, barcode sequences) is present on the surface in form of discrete clusters and can be directly used.
   b. All sequencing reactions using discrete nucleic acid clusters on a solid surface for sequencing can be used.

The advantages of the above described embodiment are the following:
1. A first adapter sequence including a molecular barcode and other functional elements needed for downstream applications (e.g. amplification) is introduced into the nucleic acids during the capturing process. The introduction of the molecular barcode does not interfere with the amplification process.
2. A second adapter sequence has to be ligated to the target nucleic acids. Therefore only one of the two adaptors is ligated to the template.
3. The process prevents the ligation of the same adapter sequence at both ends of the nucleic acids and the amplification of such nucleic acids during the amplification process, a common problem in nucleic acid preparation for sequencing.
4. A maximum density of surface bound oligonucleotides can be used for capturing of nucleic acids due to the dual function of these oligonucleotides
5. A maximum density of surface bound oligonucleotides can be used for amplification of captured target nucleic acids due to the dual function of these oligonucleotides
6. The captured and amplified sequences are forming clusters that can be directly used for sequencing on the same surface.
7. Several capturing processes can be done on one surface to accumulate different compositions of sequences from different complex nucleic acid mixtures with different sample barcodes for differentiation. This way the full capacity of the surface can be used even if the efficiency of a single capturing process is low.

### Kits

Reagents necessary to perform the method of the invention may be comprised in kits suitable for enriching template nucleic acids.

In some embodiments, the invention relates to kits for enriching nucleic acids, wherein the kit comprises
i. A mixture of oligonucleotides that can be immobilized to a solid support and contain at least two functional sequence elements.
ii. A polymerase for the extension process.
iii. An enzyme with proof reading activity for the 3' truncation and amplification process.
iv. A ligase for the ligation of adapter sequences.

The kit may further include:
v. A solid support
vi. One or several wash buffers
vii. A buffer for the ligation process
viii. A buffer for the hybridization process
ix. A buffer for the 3' truncation and amplification process
x. One or several different adapter nucleic acids, differing in the sample barcode

In some embodiments, kits providing reagents for the method described, may comprise the following components:
i. A mixture of oligonucleotides immobilized to a solid support and containing at least two functional sequence elements.
ii. A polymerase for the extension process.
iii. An enzyme with proof reading activity for the 3' truncation and amplification process.
iv. A ligase for the ligation of the adapter sequences.

Further components of the kit could be:
v. One or several wash buffers
vi. A buffer for the ligation process
vii. A buffer for the hybridization process
viii. A buffer for the 3' truncation and amplification process
ix. One or several different adapter nucleic acids, differing in the sample barcode

In some embodiments, kits providing reagents for the method described, may comprise the following components:
i. A mixture of oligonucleotides that can be immobilized to a solid support and contain at least two functional sequence elements.
ii. A polymerase for the extension process.
iii. An enzyme with proof reading activity for the amplification process.
iv. A ligase for the ligation of the adapter sequences.
v. A single strand specific 3'-5'exonuclease for the 3' truncation

The kit may further include:
vi. A solid support
vii. One or several wash buffers
viii. A buffer for the ligation process
ix. A buffer for the hybridization process
x. A buffer for the amplification process
xi. A buffer for the digestion process
xii. A buffer for the denaturation process
xiii. A buffer for the hybridization of the protecting oligonucleotides
xiv. Buffers for the sequencing reaction
xv. One or several mixtures of protecting oligonucleotides
xvi. One or several different adapter nucleic acids, differing in the sample barcode

In other embodiments, kits providing reagents for the method described, may comprise the following components:
i. A mixture of oligonucleotides that can be immobilized to a solid support and contain at least two functional sequence elements.
ii. A polymerase for the extension process.
iii. An enzyme with proof reading activity for the amplification process.
iv. A ligase for the ligation of the adapter sequences.
v. A single strand specific 3'-5'exonuclease for the 3' truncation

The kit may further include:
vi. A solid support
vii. One or several wash buffers
viii. A buffer for the ligation process
ix. A buffer for the hybridization process
x. A buffer for the amplification process
xi. A buffer for the digestion process
xii. A buffer for the denaturation process
xiii. A buffer for the hybridization of the protecting oligonucleotides
xiv. Buffers for the sequencing reaction
xv. One or several mixtures of protecting oligonucleotides
xvi. One or several different adapter nucleic acids, differing in the sample barcode

### EXAMPLES

The method of the invention is illustrated in the following examples.

**Table 1: Oligonucleotides used for examples 1 to 3**

| The first sequence element of primer 1 (element comp 1) is underlined, the second sequence element of primer 1 (element comp 2) is bold. | | |
|---|---|---|
| **Oligonucleotide** | **Comment** | **Sequence** |
| Primer 1 | Primer with two different sequence elements for specific capturing; immobilized onto surface | BIOTIN- AAAAAAAAAAAAAAAAAAAAAAAAAAAAAAAA AAAA*C*CAACATCCCACGCCTAGTCCCCA GAACATGGGCTTTCTTG (SEQ ID NO: 1) |
| Primer 2 | Primer complementary to the first sequence element | GGACTAGGCGTGGGATGTT (SEQ ID NO: 2) |
| Primer 3 | qPCR-Primer for template 1 | GCAGAGACAAGAGGATGGCT (SEQ ID NO: 3) |
| Primer 4 | qPCR-Primer for template 1 | AACATCCCACGCCTAGTCC (SEQ ID NO: 4) |
| Primer 5 | qPCR-Primer for template 2 | GCCAGATTCCAGATGAGGAC (SEQ ID NO: 5) |
| Primer 6 | qPCR-Primer for template 2 | CCAGAACATGGGCTTTCTTG (SEQ ID NO: 6) |
| Template nucleic acid 1 | Template that can be specifically captured by the first sequence element | CAACAACAACAACAACAAGGGCAGAGACA AGAGGATGGCTAGGCGAGGAGCTCCAGTC GGGGGGTGCCCAGGTCAGTGGATCCCCT CTCCACCCTGGCCTACCTGGTCGCCATGG GCGTGCCTGCCAATGGTGATGGGCTTGGT CCAGCCAGGGACTAGGCGTGGGATGTT |
| | | (SEQ ID NO: 7) |
| Template nucleic acid 2 | Template that can be specifically captured by the second sequence element | CAACAACAACAACAACAAGGGCCAGATTC CAGATGAGGACATCACAGCTTCCAGTCAG TGGTCAGAGTCCACAGCTGCCAAATATGG AAGGTGAGGATGGTTACATCAAGAAAGCC CATGTTCTGG (SEQ ID NO: 8) |
| Template nucleic acid 3 | Template that cannot be specifically captured by a sequence element | GAGACGCTGGAGTACAAACGCCAGCTGGC TGCACTTGGCGACAAGGTTACGTATCAGG AGCGCCTGAACGCGCTGGCGCAGCAGGC GGATAAATTCGCACAGCAGCAACGGGCAA AACGGGCCGCCATTGATGCGAAAAGCCGG GGGCTGACTGACCGGCAGGCAGAACGGG AAGCCACGGAACAGCGCCTGAAGGAACAG TATGGCGATAATCCGCTGG (SEQ ID NO: 9) |

| | | |
|---|---|---|
| * means phosphorothioate bond | | |

### Example 1:

### Background of the experiment:

Proof of specific functionality of the 3'-sequence element without functionality of the 5'sequence element before the partial digestion (3' truncation) of the oligonucleotides immobilized to the surface.

### Experimental details:

### (A) Immobilization of primer to the surface:

Streptavidin Coated High Capacity Plates (Pierce) were loaded with primer 1, consisting of a poly-A-Spacer and 2 consecutive sequence elements (comp 1 and comp 2) complementary to different templates (template nucleic acids 1 and 2 respectively). After binding of primer 1 to the surface, the wells were washed to remove all primers that were not bound by the Streptavidin-Biotin interaction.

### (B) Blocking of a sequence element within primer 1:

The wells were incubated with an excess of primer 2 which is an oligonucleotide complementary to element comp 1 of primer 1, resulting in a protected double-stranded region at the element comp 1. The hybridization of primer 2 was done with a temperature gradient (75°C for 3 min, 70°C for 3 min, 65°C for 3 min, 60°C for 10 min). The hybridization mixture comprises 50 mM Tris-HCI (pH8,8), 100 mM NaCl, 15 mM MgCl₂, 55% Polyethylene Glycol 300 and 2 µM primer 2. After the hybridization was finished, the surface was washed to eliminate non-hybridized oligonucleotides.

### (C) Selective hybridization of template nucleic acid and extension:

A specific hybridization of template DNA and extension reaction was performed at 45°C for 40 min with 400 pg of a mixture of templates (100 pg template 1 complementary to element comp 1, 100 pg template 2 complementary to element comp 2, 200 pg template 3 not complementary to any region of primer 1). The hybridization and extension reaction mixture comprises 20 mM Tris-HCI (pH8.8), 2 mM MgSO₄, 10 mM (NH₄)₂SO₄, 0.1% Triton X-100, 10mM KCI, 0.25 mM dNTP, 1 µg/µl BSA, 0.48 U/µl Bst Polymerase and 8 pg/µl of the mixture of templates. After the hybridization and extension reaction was finished, the surface was washed to eliminate non hybridized template nucleic acid.

### (D) Truncation of the 3' located sequence element of the immobilized primer:

The partial digestion (3' truncation) of primer 1 was performed at 37°C for 20 min followed by 5 min incubation at 65°C and again 20 min at 37°C. The digestion mixture (pH9.5) comprises 67 mM Glycine-KOH, 6.7 mM MgCI2, 10 mM β-ME and 0.68 U/µl Exonuclease I. After the digestion was finished the surface was washed several times to eliminate unspecific binding products. The washing procedure included a stringent washing step (for 5 min at 60°C) with a wash buffer comprising 50% DMSO, 0.012% Tween 20, 125 mM NaCl, 10 mM MgCl₂ and 50 mM Tris-HCI (pH7.5).

A second hybridization and extension reaction was performed at 45°C for 40 min without any template to mimic a second round of specific capturing of template (for reasons of comparability). The hybridization and extension reaction mixture comprises 20 mM Tris-HCI (pH8.8), 2 mM MgSO₄, 10 mM (NH₄)₂SO₄, 0.1% Triton X-100, 10mM KCI, 0.25 mM dNTP, 1 µg/µl BSA, 0.48 U/µl Bst Polymerase and 0 pg/µl of the mixture of templates. After the hybridization was finished the surface was washed several times to eliminate unspecific binding products. The washing procedure was done as described before (also including the stringent washing step). In order to elute the nucleic acids selectively bound to the primers immobilized to the surface, the surfaces were treated with 50 µl reconstituted DLB solution (REPLI-g Single Cell Kit, QIAGEN) for 10 min. Thereafter, 50 µl of Stop Solution (REPLI-g Single Cell Kit, QIAGEN) was added to the surfaces. 100 µl of the eluate was transferred to a new microcentrifuge tube. 2 µl of a 1:10 diluted eluate were used for 20 µl quantitative real-time PCR reactions. The real-time PCR reactions were setup by QuantiFast SybrGreen reagents (QIAGEN) and primer 3 and 4 (for template 1) or primer 5 and 6 (for template 2) as described in handbook. In a separate Real-time PCR reaction within the same run 0.1, 1, 10,100 and 1000 fg DNA were used as a standard for quantification of the templates after hybridization.

### Results:

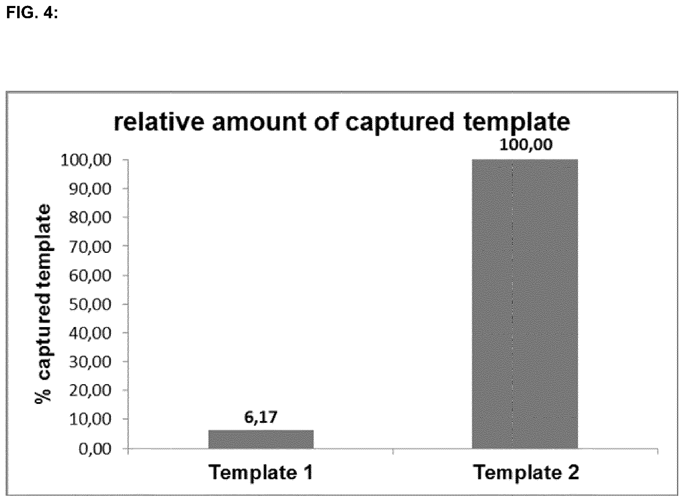

Only template 2 that hybridized to the 3'-sequence element comp 2, was captured effectively. Just a minor fraction of template 1, hybridizing to the 5'-sequence element comp 1, is detectable after hybridization (see Figure 4).

### Conclusion:

During the process, the sequence element comp 1 complementary to template 1 was blocked (by primer 2 concerning the hybridization and by the consecutive sequence element comp 2 concerning the extension) and sequence element comp 2 complementary to template 2 was accessible for template hybridization (single stranded) and extension (existing 3'-OH end). In consequence, the most 3'-sequence element comp 2 can be used before the partial digestion (3' truncation) of the oligonucleotides immobilized to the surface. Therefore, a specific capturing of the respective template (template 2) is possible without unintentional capturing of templates (like template 1) that are complementary to other sequence elements (like element comp 1).

### Example 2:

### Background of the experiment:

Proof of activation of the function of the 5'-sequence element and deactivation of the function of the 3'-sequence element by partial digestion (3' truncation) of the oligonucleotides immobilized to the surface.

### Experimental details:

### (A) Immobilization of primer to the surface:

Streptavidin Coated High Capacity Plates (Pierce) were loaded with primer 1, consisting of a poly-A-Spacer and 2 consecutive sequence elements (comp 1 and comp 2) complementary to different templates (template nucleic acids 1 and 2 respectively). After binding of primer 1 to the surface, the wells were washed to remove all primers that were not bound by the Streptavidin-Biotin interaction.

### (B) Blocking of a sequence element within primer 1:

The wells were incubated with an excess of primer 2 which is an oligonucleotide

complementary to element comp 1 of primer 1, resulting in a protected double-stranded region at the element comp 1. The hybridization of primer 2 was done with a temperature gradient (75°C for 3 min, 70°C for 3 min, 65°C for 3 min, 60°C for 10 min). The hybridization mixture comprises 50 mM Tris-HCI (pH8,8), 100 mM NaCl, 15 mM MgCl₂, 55% Polyethylene Glycol 300 and 2 µM primer 2. After the hybridization was finished, the surface was washed to eliminate non-hybridized oligonucleotides.

### (C) Selective hybridization of template nucleic acid and extension:

A specific hybridization and extension reaction was performed at 45°C for 40 min without any template to mimic a first round of specific capturing of template (for reasons of comparability). The hybridization and extension reaction mixture comprises 20 mM Tris-HCI (pH8.8), 2 mM MgSO₄, 10 mM (NH₄)₂SO₄, 0.1% Triton X-100, 10mM KCI, 0.25 mM dNTP, 1 µg/µl BSA, 0.48 U/µl Bst Polymerase and 0 pg/µl of the mixture of templates. After the hybridization and extension reaction was finished, the surface was washed to eliminate non hybridized template nucleic acid.

### (D) Truncation of the 3'located sequence element of the immobilized primer:

The partial digestion (3' truncation) of primer 1 was performed at 37°C for 20 min followed by 5 min incubation at 65°C and again 20 min at 37°C. The digestion mixture (pH9.5) comprises 67 mM Glycine-KOH, 6.7 mM MgCI2, 10 mM β-ME and 0.68 U/µl Exonuclease I. After the digestion was finished the surface was washed several times to eliminate unspecific binding products. The washing procedure included a stringent washing step (for 5 min at 60°C) with a wash buffer comprising 50% DMSO, 0.012% Tween 20, 125 mM NaCl, 10 mM MgCl₂ and 50 mM Tris-HCI (pH7.5).

A second hybridization and extension reaction was performed at 45°C for 40 min with 400 pg of a mixture of templates (100 pg template 1 complementary to element comp 1, 100 pg template 2 complementary to element comp 2, 200 pg template 3 not complementary to any region of primer 1). The hybridization and extension reaction mixture comprises 20 mM Tris-HCI (pH8.8), 2 mM MgSO₄, 10 mM (NH₄)₂SO₄, 0.1% Triton X-100, 10mM KCI, 0.25 mM dNTP, 1 µg/µl BSA, 0.48 U/µl Bst Polymerase and 8 pg/µl of the mixture of templates. After the hybridization was finished the surface was washed several times to eliminate unspecific binding products. The washing procedure was done as described before (also including the stringent washing step). In order to elute the nucleic acids from the surface, the surfaces were treated with 50 µl reconstituted DLB solution (REPLI-g Single Cell Kit, QIAGEN) for 10 min. Thereafter, 50 µl of Stop Solution (REPLI-g Single Cell Kit, QIAGEN) was added to the surfaces. 100 µl of the eluate was transferred to a new microcentrifuge tube. 2 µl of a 1:10 diluted eluate were used for 20 µl quantitative real-time PCR reactions. The real-time PCR reactions were setup by QuantiFast SybrGreen reagents (QIAGEN) and primer 3 and 4 (for template 1) or primer 5 and 6 (for template 2) as described in handbook. In a separate Real-time PCR reaction within the same run 0.1, 1, 10,100 and 1000 fg DNA were used as a standard for quantification of the templates after hybridization.

### Results:

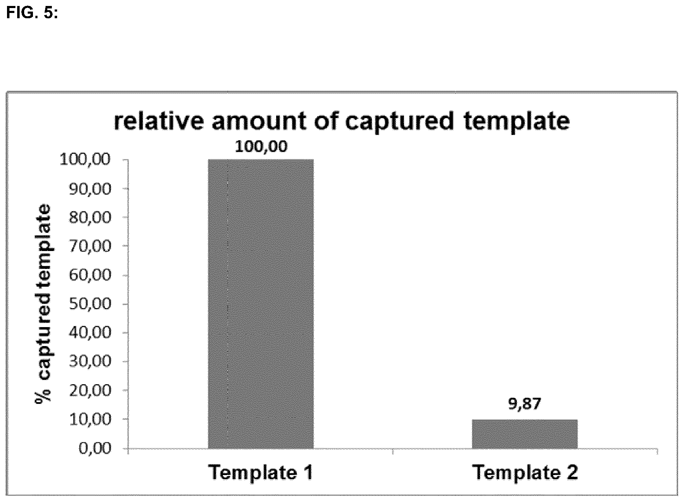

Only template 1, hybridizing to the 5'-sequence element comp 1 was captured effectively. Just a fraction of template 2, hybridizing to the 3'-sequence element comp 2, is detectable after hybridization (see Figure 5).

### Conclusion:

After the partial digestion, only the functionality of the 5'-sequence element comp 1 of primer 1 can be used for hybridization and extension. The functionality of the 5'sequence element comp 1 is activated by the partial digestion, while the functionality of the 3'-element comp 2 (complementary to template 2) is lost. Therefore the partial digestion results in specific activation and deactivation of the functions of certain consecutive sequence elements.

### Example 3:

### Background of the experiment:

Proof that the functionalities of both sequence elements of the oligonucleotides immobilized to the surface can be used consecutively by the utilization of the partial digestion (3' truncation).

### Experimental details:

### (A) Immobilization of primer to the surface:

Streptavidin Coated High Capacity Plates (Pierce) were loaded with primer 1, consisting of a poly-A-Spacer and 2 consecutive sequence elements (comp 1 and comp 2) complementary to different templates (template nucleic acids 1 and 2 respectively). After binding of primer 1 to the surface, the wells were washed to remove all primers that were not bound by the Streptavidin-Biotin interaction.

### (B) Blocking of a sequence element within primer 1:

The wells were incubated with an excess of primer 2 which is an oligonucleotide

complementary to element comp 1 of primer 1, resulting in a protected double-stranded region at the element comp 1. The hybridization of primer 2 was done with a temperature gradient (75°C for 3 min, 70°C for 3 min, 65°C for 3 min, 60°C for 10 min). The hybridization mixture comprises 50 mM Tris-HCI (pH8,8), 100 mM NaCl, 15 mM MgCl₂, 55% Polyethylene Glycol 300 and 2 µM primer 2. After the hybridization was finished, the surface was washed to eliminate non-hybridized oligonucleotides.

### (C) Selective hybridization of template nucleic acid and extension:

A specific hybridization of template DNA and extension reaction was performed at 45°C for 40 min with 400 pg of a mixture of templates (100 pg template 1 complementary to element comp 1, 100 pg template 2 complementary to element comp 2, 200 pg template 3 not complementary to any region of primer 1). The hybridization and extension reaction mixture comprises 20 mM Tris-HCI (pH8.8), 2 mM MgSO₄, 10 mM (NH₄)₂SO₄, 0.1% Triton X-100, 10mM KCI, 0.25 mM dNTP, 1 µg/µl BSA, 0.48 U/µl Bst Polymerase and 8 pg/µl of the mixture of templates. After the hybridization and extension reaction was finished, the surface was washed to eliminate non hybridized template nucleic acid.

### (D) Truncation of the 3'located sequence element of the immobilized primer:

The partial digestion (3' truncation) of primer 1 was performed at 37°C for 20 min followed by 5 min incubation at 65°C and again 20 min at 37°C. The digestion mixture (pH9.5) comprises 67 mM Glycine-KOH, 6.7 mM MgCI2, 10 mM β-ME and 0.68 U/µl Exonuclease I. After the digestion was finished the surface was washed several times to eliminate unspecific binding products. The washing procedure included a stringent washing step (for 5 min at 60°C) with a wash buffer comprising 50% DMSO, 0.012% Tween 20, 125 mM NaCl, 10 mM MgCl₂ and 50 mM Tris-HCI (pH7.5).

A second hybridization and extension reaction was performed at 45°C for 40 min with 400 pg of a mixture of templates (100 pg template 1 complementary to element comp 1, 100 pg template 2 complementary to element comp 2, 200 pg template 3 not complementary to any region of primer 1). The hybridization and extension reaction mixture comprises 20 mM Tris-HCI (pH8.8), 2 mM MgSO₄, 10 mM (NH₄)₂SO₄, 0.1% Triton X-100, 10mM KCI, 0.25 mM dNTP, 1 µg/µl BSA, 0.48 U/µl Bst Polymerase and 8 pg/µl of the mixture of templates. After the hybridization was finished the surface was washed several times to eliminate unspecific binding products. The washing procedure was done as described before (also including the stringent washing step). In order to elute the nucleic acids from the surface, the surfaces were treated with 50 µl reconstituted DLB solution (REPLI-g Single Cell Kit, QIAGEN) for 10 min. Thereafter, 50 µl of Stop Solution (REPLI-g Single Cell Kit, QIAGEN) was added to the surfaces. 100 µl of the eluate was transferred to a new microcentrifuge tube. 2 µl of a 1:10 diluted eluate were used for 20 µl quantitative real-time PCR reactions. The real-time PCR reactions were setup by QuantiFast SybrGreen reagents (QIAGEN) and primer 3 and 4 (for template 1) or primer 5 and 6 (for template 2) as described in handbook. In a separate Real-time PCR reaction within the same run 0.1, 1, 10,100 and 1000 fg DNA were used as a standard for quantification of the templates after hybridization.

### Results:

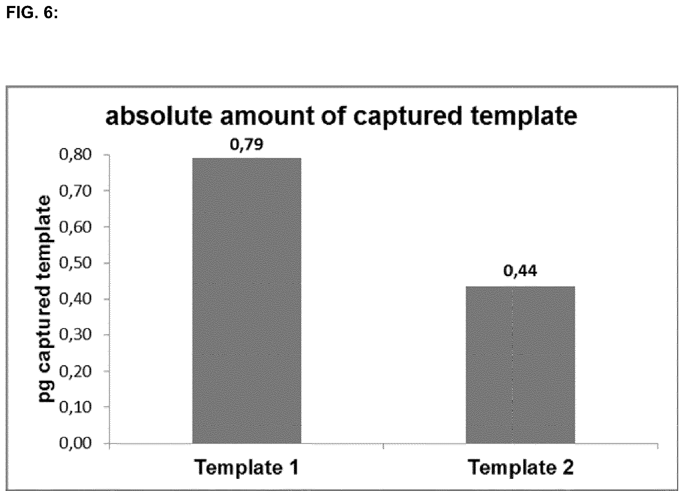

Template 1 and template 2 were captured effectively by two rounds of specific hybridization and extension, separated by the partial digestions. The binding efficiency of template 1 seems to be a little bit higher than the binding efficiency of template 2, but the amounts of captured templates are both in the expected range (see Figure 6).

### Conclusion:

The results show that the functionalities of both sequence elements can be used subsequently by the use of the partial digestion. The 3'-sequence element comp 2 can be used before the partial digestion to specifically capture template 2 and the 5'sequence element comp 1 can be used after partial digestion to specifically capture template 1. An oligonucleotide with consecutive sequence elements having different functionalities is therefore possible.

### Example 4:

**Table 2: Oligonucleotides used for example 4 /3ddC/ is the symbol for a dideoxy-Cytosine, sequence element with the functionality for the π-Amp-reaction (π-Amp-element) in bold, 3'-sequence-element complementary to template nucleic acid 1 (comp 1) underlined, 3'-sequence-element not complementary to template nucleic acid 1 (no-comp 1) italic**

| **Oligonucleotide** | **Comment** | **Sequence** |
|---|---|---|
| Primer 1 | Primer with two different sequence elements; immobilized onto surface | Biotin- AAAAAAAAAAAAAAAAAAAAAAAAAAAAA AAAAAACCCCAGAACATGGGCTTTCTTGCC/ 3ddC/ (SEQ ID NO: 10) |
| Primer 2 | π-Amp-primer; immobilized onto surface | Biotin-AAA AAA AAA AAA AAA AAA AAA AAA AAA AAA AAA AA*C C/3ddC/ (SEQ ID NO: 11) |
| Primer 3 | Primer with two different sequence elements; immobilized onto surface | Biotin- AAAAAAAAAAAAAAAAAAAAAAAAAAAAAAAA AAAAAACCAACATCCCACGCCTAGTCCCC/3 ddC/ (SEQ ID NO: 12) |
| Primer 4 | qPCR-Primer | GCCAGATTCCAGATGAGGAC (SEQ ID NO: 5) |
| Primer 5 | qPCR-Primer | CCAGAACATGGGCTTTCTTG (SEQ ID NO: 6) |
| Template nucleic acid 1 | Template that can be specifically captured by the 3'sequence element comp 1 | CAACAACAACAACAACAAGGGCCAGATTCCA GATGAGGACATCACAGCTTCCAGTCAGTGG TCAGAGTCCACAGCTGCCAAATATGGAAGG TGAGGATGGTTACATCAAGAAAGCCCATGTT CTGG (SEQ ID NO: 8) |

| | | |
|---|---|---|
| * means phosphorothioate bond | | |

### Background of the experiment:

Proof that an oligonucleotide immobilized to the surface can have (at least) one sequence element for capturing a template and another sequence element for amplifying the captured template. Proof that the amplification reaction and the partial digestion (3' truncation) can take place in the same reaction in parallel. Proof that a partial digestion (to activate the Amplification sequence element) is needed for efficient amplification if no functional primers for the amplification are provided from the very beginning on.

### Experimental details:

Primer 1 and primer 3 comprise a 5'-sequence-element providing the functionality for the π-Amp-reaction (π-Amp element) and a 3'-sequence-element for specific capturing of a target. While primer 1 comprises the 3'-sequence element comp 1 that is complementary to the template nucleic acid 1 used in this experiment, primer 3 comprises a 3'sequence-element not complementary to template 1 (element no-comp 1).
Primer 2 only consists of the 5'- π-Amp element and therefore is the standard oligonucleotide for a π-Amp-reaction.

### (A) Immobilization of primer mixes to the surface:

Streptavidin Coated High Capacity Plates (Pierce) were loaded with one of the following mixes:

### 1.) π-Amp surface-mix (primer 1 diluted 1:10 in primer 2, reactions 1 and 2):

The mixture of primer 1 (10%) and primer 2 (90%) generates a surface where most of the immobilized oligonucleotides function as π-Amp-primers (primer 2) supporting the π-Amp reaction and only a minor fraction has no π-Amp functionality but specifically captures a template (here template 1) during the hybridization and extension reaction (primer 1). This setup is close to the reaction conditions in a normal π-Amp-reaction.

### 2.) Capturing-surface-mix (primer 1 diluted 1:10 in primer 3, reactions 3, 4 and 5):

The mixture of primer 1 (10%) and primer 3 (90%) generates a surface where none of the immobilized oligonucleotides comprises accessible π-Amp elements necessary for a π-Amp-reaction.Primer 1 as well as primer 3 posses 3'-elements for capturing of specific templates (comp 1 for template 1 and no-comp 1 for some other template). This setup mimics a surface that has a high density of oligonucleotides for specific capturing of different templates but does not support an amplification reaction.

After binding the wells were washed to remove all primers that were not bound by the Streptavidin-Biotin interaction.

### (B) Selective hybridization of template nucleic acid and extension:

A specific hybridization and extension reaction was performed at 45°C for 40 min with 100 pg of template 1. Template 1 is specifically captured by the 3'-sequence element comp 1 of primer 1. The hybridization and extension reaction mixture comprises 20 mM Tris-HCI (pH8.8), 2 mM MgSO₄, 10 mM (NH₄)₂SO₄, 0.1% Triton X-100, 10mM KCI, 0.25 mM dNTP, 1 µg/µl BSA, 0.48 U/µl Bst Polymerase and 2 pg/µl of template 1. After the hybridization and extension reaction was finished, the surface was washed to eliminate non hybridized template nucleic acid.

### (C) π-amplification with different reaction conditions:

The π-amplification was performed for 60 min at 57°C in an amplification reaction mixture comprising 20 mM Tris-HCI (pH8.8), 1.25 mM MgSO₄, 10 mM (NH₄)₂SO₄, 0.1% Triton X-100, 10mM KCI, 0.25 mM dNTP, 1 µg/µl BSA, 1 µM Primer PEkurzGG, 25% Dextrane, 0.48 U/µl Bst Polymerase, and 0.01 U/µl Pfu Polymerase (used for deblocking of 3'-terminal blocked primer). In the reactions with additional digestion activity the concentration of Pfu Polymerase was increased by 800 % resulting in a final concentration of 0,09 U/µl Pfu Polymerase. In the control reactions the amplification reaction mixture did not comprise any enzymes and no mobile primer (both needed for the amplification reaction). Modifications of the reaction are listed in Table 3.

After the amplification was finished the vials were washed to eliminate non bound amplification products. In order to elute the amplification products from the surface, the surfaces were treated with 50 µl reconstituted DLB solution (REPLI-g Single Cell Kit, QIAGEN) for 10 min. Thereafter, 50 µl of Stop Solution (REPLI-g Single Cell Kit, QIAGEN) was added to the surfaces. 100 µl of the eluate was transferred to a new microcentrifuge tube. 2 µl of a 1:10 diluted eluate were used for 20 µl quantitative real-time PCR reactions. The real-time PCR reactions were setup by QuantiFast SybrGreen reagents (QIAGEN) and primer 4 and 5 as described in handbook. In a separate Real-time PCR reaction within the same run 0.1, 1, 10, 100, 1000 or 10 000 pg DNA were used as a standard for quantification of the specific DNA after amplification.

**Table 3: Variations of the reaction**

| **Reaction** | **% Primer 1** | **% Primer 2** | **% Primer 3** | **Additional digestion activity** | **π-Amp with enzymes/mobile Primer** |
|---|---|---|---|---|---|
| 1-π-Amp | 10 | 90 | 0 | - | + |
| 2-π-Amp-Ctrl. | 10 | 90 | 0 | - | - |
| 3- π-Amp | 10 | 0 | 90 | - | + |
| 4- π-Amp +digestion | 10 | 0 | 90 | + | + |
| 5 π-Amp-Ctrl. | 10 | 0 | 90 | - | - |

### Results and Conclusions:

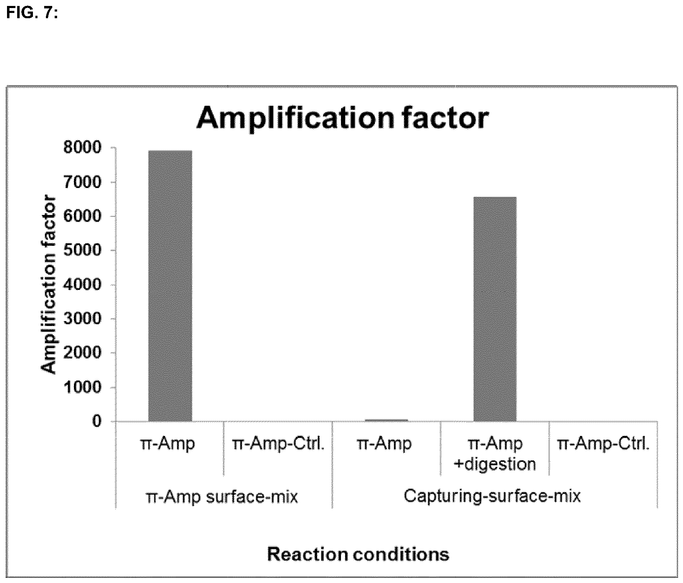

### (see Figure 7)

### Reaction 1:

The conditions in reaction 1 are very close to the conditions in a regular π-amplification. Most of the immobilized oligonucleotides (90% primer 2) do have the π-amplification functionality and the π-amplification mixture is not modified. The amplification factor under this conditions was >7900fold.

The result is expected because most of the oligonucleotides on the surface support the π-amplification. The slightly lower amplification factor is probably because of the 10% of surface bound primers without an active functional π-amp-element (primer 1).

### Reaction 2 (Control for reaction 1):

The conditions in reaction 2 are similar to the conditions in reaction 1. The only difference is the absence of enzymes and the mobile primer in the amplification mixture. This made sure that no amplification took place, in order to determine the amount of hybridized and extended template nucleic acid molecules. The amplification factor in reaction 1 can be calculated by dividing the amount of specific templates after the amplification through the amount of specific templates in reaction 2.

### Reaction 3:

The conditions in reaction 3 are not very close to a regular π-amplification because there are only immobilized primers that do not have accessible π-amp-elements needed for the amplification (primer 1 and primer 3). The π-amplification mixture is not modified. The resulting amplification factors are very low based on control reaction 5. This result is expected because of the lack of accessible π-amp-elements in primer 1 and primer 3. Without the functional elements no π-amp-reaction can occur, although the reaction mixture would allow an efficient π-amplification. The residual amplification is very likely due to the partial digestion of a minor fraction of surface bound oligonucleotides by the proof reading activity of the Pfu polymerase, which activates some of the 3'- π-amp-elements of primers 1 and 3.

### Reaction 4:

The conditions in reaction 4 are very similar to reaction 3. As in reaction 3 the surface comprises primer 1 and 3 without any accessible π-amp-elements needed for the amplification. The important difference is the modification of the π-amplification mixture. The amount of Pfu-polymerase in the π-amplification reaction is increased by 800%. The amplification factor using this π-amplification with additional digestion activity was >6500fold (based on control reaction 5), which is very close to the amplification factor of reaction 1 (reaction with a majority of accessible functional π-amp-elements on the surface). The 3'-5'-exonuclease activity of the highly concentrated Pfu-polymerase allows the efficient partial digestion of primers 1 and 3. This activates the functionality of the 3'-π-amp-elements of primers 1 and 3 by making them accessible for the π-amplification-reaction. Therefore the partial digestion for the activation of the π-amp-elements and the π-amplification take place in the same reaction in parallel.

### Reaction 5 (Control for reactions 3 and 4):

The conditions in reaction 5 are similar to the conditions in reaction 3. The only difference is the absence of enzymes and the mobile primer in the amplification mixture. This made sure that no amplification took place, in order to determine the amount of hybridized and extended template nucleic acid molecules. The amplification factor in reactions 3 and 4 can be calculated by dividing the amount of specific templates after the amplification through the amount of specific templates in reaction 5.

### General conclusions:

The experiment shows that primers with consecutive sequence elements can be used for multiple functionalities (specific capturing of target nucleic acids and amplification). The partial digestion (3' truncation) and the amplification can be done in the same reaction in parallel. Without the digestion of the 3'-sequence elements, the amplification functionality cannot be activated.

The usage of those primers allows a high density of oligonucleotides on the surface for specific capturing of template nucleic acids combined with an efficient amplification of the captured nucleic acids.

In view of the above, it will be appreciated that the invention also encompasses the following items:
1. A method for enriching template nucleic acids, wherein the method comprises:
   a) hybridizing template nucleic acids to oligonucleotides bound to a solid surface and initially comprising at least two functional sequence elements;
   b) extending the surface bound oligonucleotides hybridized to the template nucleic acids to form a double strand;
   c) optionally modifying the double-stranded nucleic acids generated in step b);
   d) 3' truncation of the surface bound oligonucleotides that have not been used in the template nucleic acid hybridization of step a); and
   e) optionally modifying the single-stranded surface bound oligonucleotides generated in step d).
2. The method according to item 1, further comprising the additional step of:
   f) hybridizing further template nucleic acids to the surface bound oligonucleotides
   or
   using functional sequence elements within surface bound oligonucleotides for a downstream application.
3. The method according to item 2, wherein the downstream application is nucleic acid amplification on the solid surface, de-coupling from solid surface, in-vitro transcription by the use of an RNA polymerase promotor within the oligonucleotide, labeling of immobilized nucleic acid by the use of a primer binding site or a molecular barcode region for identification within the oligonucleotide, sequencing, or a combination thereof.
4. The method according to any of the preceding items, wherein at least one of the functional sequence elements is a hybridization site or preferably a sequence useful for a downstream application.
5. The method according to any of the preceding items, wherein the functional sequence elements are consecutive or overlapping.
6. The method according to any of the preceding items, wherein the functional sequence elements are separated by predefined cleavage sites or generated by hybridization of protecting oligonucleotides to the surface bound oligonucleotides.
7. The method according to any one of the preceding items, wherein steps a) to c) are repeated at least once with template nucleic acids from different samples.
8. The method according to any one of items 1-6, wherein steps a) to e) are repeated at least once with template nucleic acids from the same sample or from different samples, optionally wherein the repetition(s) is (are) performed in parallel.
9. The method according to any of the preceding items, wherein the density of the surface bound oligonucleotides is between 500 - 500000 oligonucleotides / µm², more preferably 750 - 200000 oligonucleotides / µm², most preferably 1000 - 100000 oligonucleotides / µm².
10. The method according to any of the preceding items, wherein the surface bound oligonucleotides comprise 2 to 20 functional sequence elements, more preferably 2 to 10, most preferably 2 to 5.
11. The method according to any of the preceding items, wherein the length of the surface bound oligonucleotides is within the range of 4-200 nt, preferably 6-180 nt, more preferably 8-160 nt, more preferably 10-140 nt, most preferably 20-100 nt.
12. The method according to any of the preceding items, wherein all functional sequence elements of the same position within a surface bound oligonucleotide have a unique sequence or comprise 2-100000, preferably 2-50000, more preferably 2-25000, more preferably 2-10000, more preferably 2-5000, more preferably 2-2500, most preferably 2-1000 different sequences.
13. The method according to any of the preceding items, wherein the 3' truncation is achieved enzymatically or chemically.
14. The method according to any of the preceding items, wherein the double-stranded nucleic acids bound to the surface are modified by introducing barcode sequences, adding sequencing adaptors, adding a fluorophore at the terminus, incorporation of modified bases, or other modifications.
15. The method according to any of the preceding items, wherein the single-stranded oligonucleotides bound to the surface are modified by adding biotin, labeling moieties, blocking moieties, or other modifications.

## Claims

1. A method for enriching template nucleic acids, wherein the method comprises:
a) hybridizing template nucleic acids to oligonucleotides bound to a solid surface and initially comprising at least two functional sequence elements;
b) extending the surface bound oligonucleotides hybridized to the template nucleic acids to form a double strand;
c) optionally modifying the double-stranded nucleic acids generated in step b);
d) 3' truncation of the surface bound oligonucleotides that have not been used in the template nucleic acid hybridization of step a); and
e) optionally modifying the single-stranded surface bound oligonucleotides generated in step d).

2. The method according to claim 1, further comprising the additional step of:
f) hybridizing further template nucleic acids to the surface bound oligonucleotides
or
using functional sequence elements within surface bound oligonucleotides for a downstream application.

3. The method according to claim 2, wherein the downstream application is nucleic acid amplification on the solid surface, de-coupling from solid surface, in-vitro transcription by the use of an RNA polymerase promotor within the oligonucleotide, labeling of immobilized nucleic acid by the use of a primer binding site or a molecular barcode region for identification within the oligonucleotide, sequencing or another application, or a combination thereof.

4. The method according to any of the preceding claims, wherein at least one of the functional sequence elements is a hybridization site or a sequence useful for a downstream application.

5. The method according to any of the preceding claims, wherein the functional sequence elements are consecutive or overlapping.

6. The method according to any of the preceding claims, wherein the functional sequence elements are separated by predefined cleavage sites or generated by hybridization of protecting oligonucleotides to the surface bound oligonucleotides.

7. The method according to any one of the preceding claims, wherein steps a) to c) are repeated at least once with template nucleic acids from different samples.

8. The method according to any one of claims 1-6, wherein steps a) to e) are repeated at least once with template nucleic acids from the same sample or from different samples, optionally wherein the repetition(s) is (are) performed in parallel.

9. The method according to any of the preceding claims, wherein the density of the surface bound oligonucleotides is between 500 - 500000 oligonucleotides / µm², more preferably 750 - 200000 oligonucleotides / µm², most preferably 1000 - 100000 oligonucleotides / µm².

10. The method according to any of the preceding claims, wherein the surface bound oligonucleotides comprise 2 to 20 functional sequence elements, more preferably 2 to 10, most preferably 2 to 5.

11. The method according to any of the preceding claims, wherein the length of the surface bound oligonucleotides is within the range of 4-200 nt, preferably 6-180 nt, more preferably 8-160 nt, more preferably 10-140 nt, most preferably 20-100 nt.

12. The method according to any of the preceding claims, wherein all functional sequence elements of the same position within a surface bound oligonucleotide have a unique sequence or comprise 2-100000, preferably 2-50000, more preferably 2-25000, more preferably 2-10000, more preferably 2-5000, more preferably 2-2500, most preferably 2-1000 different sequences.

13. The method according to any of the preceding claims, wherein the 3' truncation is achieved enzymatically or chemically.

14. The method according to any of the preceding claims, wherein the double-stranded nucleic acids bound to the surface are modified by introducing barcode sequences, adding sequencing adaptors, adding a fluorophore at the terminus, incorporation of modified bases, or other modifications.

15. The method according to any of the preceding claims, wherein the single-stranded oligonucleotides bound to the surface are modified by adding biotin, labeling moieties, blocking moieties, or other modifications.
